# EUROPEAN PATENT APPLICATION

(11) **EP 2 332 526 A2**
(43) Date of publication of application: **15.06.2011**
(21) Application number: 11002109.4
(22) Date of filing: 20.10.2006
(51) Int. Cl.: A61K 31/137, A61K 31/165, A61K 31/167, A61K 31/365, A61K 31/397, A61K 31/445, A61K 31/455, A61K 31/4706, A61K 31/785, A61K 45/06, A61P 3/04, A61P 3/06, A61P 3/10, A61P 9/00, A61P 9/10, A61P 9/12

(54) **Combination of a renin-inhibitor and an anti-dyslipidemic agent and/or an antiobesity agent**

(30) Priority: 21.10.2005 US 728834 P
(62) Divisional of application: 06817267.5
(71) Applicant: Novartis AG, 4056 Basel (CH); Novartis Pharma GmbH, 1230 Wien (AT)
(72) Inventor: The designation of the inventor has not yet been filed
(74) Representative: Grünecker, Kinkeldey, Stockmair & Schwanhäusser Anwaltssozietät

(57) **Abstract**

The invention relates to a combination, such as a combined preparation or a pharmaceutical composition, respectively comprising a renin inhibitor, or a pharmaceutically acceptable salt thereof, and at least one therapeutic agent selected from the group consisting of (a) a specific anti-dyslipidemic agent and (b) a specific anti-obesity agent or, in each case, a pharmaceutically acceptable salt thereof.

## Description

### Background and related prior art

Disorders of lipid metabolism, or dyslipidemias, include various conditions characterized by abnormal concentrations of one or more lipids (i.e. cholesterol and triglycerides), and/or apolipoproteins (i.e., apolipoproteins A, B. C and E), and/or lipoproteins (i.e., the macromolecular complexes formed by the lipid and the apolipoprotein that allow lipids to circulate in blood, such as LDL, VLDL and IDL).

Hyperlipidemia is associated with abnormally high levels of lipids, LDL and VLDL cholesterol, and/or triglycerides. Cholesterol is mostly carried in Low Density Lipoproteins (LDL), and this component is commonly known as the "bad" cholesterol because it has been shown that elevations in LDL- cholesterol correlate closely to the risk of coronary heart disease. A smaller component of cholesterol is carried in the High Density Lipoproteins and is commonly known as the "good" cholesterol. In fact, it is known that the primary function of HDL is to accept cholesterol deposited in the arterial wall and to transport it back to the liver for disposal through the intestine. Although it is desirable to lower elevated levels of LDL cholesterol, it is also desirable to increase levels of HDL cholesterol. Generally, it has been found that increased levels of HDL are associated with lower risk for coronary heart disease (CW)). See, for example, Gordon, et al., Am. J. Med., 62, 707-714 (1977); Stampfer, et al., N. England J. Med., 325, 373 381 (1991); and Kannel, et al., Ann. Internal Med., 90, 85-91 (1979). An example of an HDL raising agent is nicotinic acid, a drug with limited utility because doses that achieve HDL raising are associated with undesirable effects, such as flushing.

Dyslipidemias were originally classified by Fredrickson according to the combination of alterations mentioned above. The Fredrickson classification includes 6 phenotypes (i.e., I, IIa, IIb, III, IV and V) with the most common being the isolated hypercholesterolemia (or type IIa) which is usually accompained by elevated concentrations of total and LDL cholesterol. The initial treatment for hypercholesterolemia is often aimed at avoiding risk factors such as obesity, and at modifying the diet to one low in fat and cholesterol, coupled with appropriate physical exercise, followed by drug therapy when LDL-lowering goals are not met by diet and exercise alone.

A second common form of dyslipidemia is the mixed or combined hyperlipidemia or type llb and III of the Fredrickson classification. This dyslipidemia is often prevalent in patients with type 2 diabetes, obesity and metabolic syndrome. In this dyslipidemia there are modest elevations of LDL-cholesterol, accompanied by more pronounced elevations of small dense LDL-cholesterol particles, VLDL and/or IDL (i.e., triglyceride rich lipoproteins), and total triglycerides. In addition, concentrations of HDL are often low.

The risk of atherosclerosis and coronary artery or carotid artery disease, and therefore the risk of having a heart attack or stroke, increases as the total cholesterol level increases. Atherosclerosis refers to a disease in which the wall of an artery becomes thicker and less elastic. In atherosclerosis, fatty material accumulates under the inner lining of the arterial wall. Atherosclerosis can affect the arteries of the brain, heart, kidneys, other vital organs, and the arms and legs. When atherosclerosis develops in the arteries that supply the brain (carotid arteries), a stroke may occur; when it develops in the arteries that supply the heart (coronary arteries), a heart attack may occur. Arteries affected with atherosclerosis lose their elasticity, an as the atheromas (patchy thickening in the inner lining of the artery) grow, the arteries narrow. With time, the atheromas collect calcium deposits, may become brittle, and may rupture. Blood may then enter a ruptured atheroma, making it larger, so that it narrows the artery even more. A ruptured atheroma also may spill its fatty contents and trigger the formation of a blood clot (thrombus). The clot may further narrow or even occlude the artery, or it may detach and float downstream where it causes an occlusion (embolism).

Causes of high cholesterol levels include a diet high in saturated fats and cholesterol; cirrhosis, poorly controlled diabetes, underactive thyroid gland, overactive pituitary gland, kidney failure, porphyria, and heredity. Causes of high triglyceride levels include excess calories in diet, severe uncontrolled diabetes, kidney failure, acute alcohol abuse, certain drugs, and heredity.

Obesity is one of the factors contributing to high levels of certain lipids, such as VLDL and LDL, as a result, the initial treatment for overweight patients with high cholesterol or triglyceride levels is to lose weight. Levels of fatty material such as cholesterol and triglycerides may also be controlled with a variety of drugs. Hydroxymethylglutaryl-coenzyme A (HMG-CoA) reductase inhibitors block the synthesis of cholesterol and enhance the removal of low density lipoproteins from the bloodstream.

Obesity is a common and chronic condition whose prevalence has increased steadily in advanced nations. Though the molecular pathways regulating energy balance are beginning to be illuminated, the causes of obesity remain elusive. In part, this reflects the fact that obesity is a heterogeneous group of disorders. At one level, the pathophysiology of obesity seems simple: a chronic excess of nutrient intake relative to the level of energy expenditure. However, due to the complexity of the neuroendocrine and metabolic systems that regulate energy intake, storage, and expenditure, it has been difficult to quantitate all the relevant parameters (e.g., food intake and energy expenditure) over time in human subjects. Obesity contributes to a myriad of health problems, including type 2 diabetes mellitus, hypertension, congestive heart failure, lipid disorders, arthritis, and some cancers. Obesity and related conditions contribute to nearly 300 000 annual deaths in the United States. Unfortunately, obesity is not well understood.

Obesity is important as a risk factor of the onset of diseases represented by geriatric diseases from hygienic and cosmetic viewpoints. Harmful influences of obesity have been recognized for a long time in advanced nations. Agents for preventing and/or treating obesity which have been developed until now have side effects or produce unsatisfactory effects. Despite short-term benefits, medication-induced weight loss is often associated with rebound weight gain after the cessation of drug use, side effects from the medications, and the potential for drug abuse. Given the need for effective therapies, many possible compounds and combinations have been evaluated. For instance when fenfluramine was administered together with phentermine, as "fen-phen," the combination was widely used based on controlled trials that demonstrated modest but definite efficacy. However, the risk of primary pulmonary hypertension was increased up to 20-fold in association with this treatment. The FDA withdrew approval of the fen-phen combination in 1997 when reports suggested an association with right- and left-sided valvular heart disease.

Thus there still exists a need for more effective anti-dyslipidemic and anti-obesity combinations with less or no side effects such as described herein and lower toxicity.

Besides genetic predisposition, obesity and dyslipidemia are the most important risk factors for the development cardiovascular disorders. Even modest weight reduction can improve e.g. blood pressure control in overweight subjects. Thus, there is furthermore a need for a combination which is also effective for treating or preventing certain cardiovascular disorders with less or no side effects such as described herein e.g. weight gain and lower toxicity. Particularly, there is a need of new combinations for the treatment and/or prevention of cardiovascular disorders, dyslipidemia or obesity and showing furthermore beneficial effects on diseases and conditions associated with cardiovascular disorders, dyslipidemia or obesity.

High blood pressure becomes increasingly difficult to treat when patients present with additional co-morbidities such as diabetes, obesity, dyslipidemia or metabolic disturbances. To achieve target blood pressure goals in patients with coexistent risk factors or conditions, multi-drug therapy is often required. If blood pressure or other co-morbidities are inadequately modified, the patient is at greater risk of serious adverse events such as myocardial infarction, stroke and progressive organ damage.

Moreover, while renin angiotensin system (RAS) blockade, either with the use of angiotensin converting enzyme inhibitors (ACEi) or with angiotensin receptor blockers (ARBs) has proven to be a very effective means of lowering elevated blood pressure, many patients, for example, obese or overweight individuals or dyslipidemic patients, may require additional therapeutic interventions to achieve specific target blood pressure goals. Furthermore, obese or overweight patients or dyslipidemic patients may need treatment with drugs designed specifically to interrupt key pathways contributing to this metabolic phenotype.

There is accumulating evidence that obese subjects have an increased risk of cardiovascular and metabolic diseases (Montani JP, Antic V, Yang Z. Pathways from obesity to hypertension: from the perspective of a vicious triangle. Internat J Obesity 26(Suppl 2):S28-S38, 2002). Also, a strong correlation between body weight and blood pressure has been demonstrated in humans (Jones DW, Kim JS, Andrew ME, Kim SJ, Hong YP. Body mass index and blood pressure in Korean men and women: the Korean National Blood Pressure Survey. J Hypertens 12:1433-1437, 1994). In short-term studies, it has been shown that weight loss in overweight or obese human subjects leads to a reduction in both systolic and diastolic blood pressure (Aucott L, Poobalan A, Smith WCS, Avenell A, Jung R, Broom J. Effects of weight loss in overweight/obese individuals and long-term hypertension outcomes. Hypertension 45:1035-1041, 2005). The precise mechanisms underlying this relationship are not known, however, a clear association between weight gain and activation of the sympathetic nervous system has been shown (Masuo K, Mikami H, Ogihara T, Tuck ML. Weight gain-induced blood pressure elevation. Hypertension 35:1135-1140, 2000). Increased sympathetic activity results in vasoconstriction and sodium retention, two factors that directly contribute to a rise in systemic blood pressure. In an animal model of diet-induced obesity in the dog, weight gain results in an increase in blood pressure, heart rate, and plasma insulin (Hall JE, Brands MW, Dixon WN, Smith MJ Jr. Obesity-induced hypertension. Renal function and systemic hemodynamics. Hypertension 22:292-299, 1993). These results suggest that a similar cause and effect relationship may exist in animals and in humans and thus allows for the study of this set of conditions in appropriate animal species. Several additional factors also may contribute to the linkage seen between weight gain and blood pressure in animals and in man including leptin, free fatty acids, and insulin. Leptin and free fatty acids rise progressively with increasing adiposity and are released by visceral adipocytes. These mediators may act alone or in concert to increase sympathetic tone and vasoconstriction, thereby leading to an increase in blood pressure (Montani et al., 2002). Adipose tissue can be considered an endocrine organ, whereby release of leptin can have profound effects within the central nervous system to induce satiety and activate the sympathetic nervous system (Pantanetti P, Garrapa GGM, Mantero F, Boscaro M, Faloia E, Venarucci D. Adipose tissue as an endocrine organ? A review of recent data related to cardiovascular complications of endocrine dysfunctions. Clin Exper Hypertens 26(4):387-398, 2004). In obese humans, leptin is elevated in plasma yet these individuals do not appear to have a normal satiety response to this hormone. The concept of selective leptin resistance has been introduced to explain the phenomenon whereby the hypothalamus becomes unresponsive to the satiety effects of leptin but the central nervous system retains full reactivity to the stimulation of the sympathetic nervous system (SNS). Consequently, the obese or overweight phenotype lingers due to the inability of leptin to invoke a satiety response. Additionally, chronic over-activity of the SNS persists and leads to an increase in systemic blood pressure (Mark AL, Correia MLG, Rahmouni K, Haynes WG. Selective leptin resistance: a new concept in leptin physiology with cardiovascular implications. J Hypertens 20:1245-1250, 2002). Thus, human obesity is considered by some to be a leptin-resistant state. The model of the Agouti yellow obese mouse may mimic this phenotype (Correia MLG, Haynes WG, Rahmouni K, Morgan DA, Sivitz WI, Mark AL. The concept of selective leptin resistance. Diabetes 51:439-442, 2002).

Recently, it has been demonstrated that adipose tissue contains all of the components of the RAS (Goossens GH, Blaak EE, van Baak MA. Possible involvement of the adipose tissue renin-angiotensin system in the pathophysiology of obesity and obesity-related disorders. Obesity Reviews 4:43-55, 2003). Thus, the RAS contained in its entirety within the adipocyte may provide an important link between a major cardiovascular control system and obesity and obesity-related diseases. A high fat diet in rodents leads to increased generation of angiotensinogen and angiotensin II in adipocytes. Angiotensin II promotes adipocyte growth. Angiotensin II, either adipocyte-derived or formed in the plasma can have profound effects on fat cells directly or in distal cell types accessible from the circulation. Clearly, angiotensin II can result in a potent vasoconstrictor effect and sodium retention to increase arterial blood pressure. The findings relating components of the RAS within and/or released from adipocytes, have been equivocal in animal models and in humans (Engeli S, Schling P, Gorzelniak K, Boschmann M, Janke J, Ailhaud G, Teboul M, Massiera F, Sharma AM. The adipose-tissue renin-angiotensin-aldosterone system: role in the metabolic syndrome? Internat J Biochem Cell Biol 35:807-825, 2003).

Although the association between body weight, dyslipidemia and blood pressure is closely linked, the assignment of specific mechanisms underlying this relationship have been more difficult to prove since investigations have relied on several species, including man and the use of various animal models, cell systems and assay conditions.

Therefore, an object of the present invention is to provide more effective anti-dyslipidemic, anti-obesity and/or compositions to treat cardiovascular disorders and new therapeutic methods with less or no side effects and lower toxicity for treating or preventing cardiovascular disorders, dyslipidemia or obesity, and conditions associated therewith.

### Summary of the present invention

In this application, the following effects were surprisingly found:
- A potentiation of the blood pressure lowering and weight loss with the use of a renin inhibitor when given in combination with a drug used for weight loss (also known as an anti-obesity agent).
- A potentiation of the blood pressure lowering and lowering of the lipid levels with the use of a renin inhibitor when given in combination with a drug used for lowering lipid levels (also known as an anti-dyslipidemic agent)

This is an unexpected finding in that the components of the combination interact in a complementary manner to elicit effects that can not be achieved by any single agent when given alone.

The present invention relates to a combination, such as a combined preparation or a pharmaceutical composition, respectively, comprising a renin inhibitor, or a pharmaceutically acceptable salt thereof, and at least one therapeutic agent selected from the group consisting of
(a) an anti-dyslipidemic agent selected from the group consisting of a bile acid sequesterant, a cholesterol absorption inhibitor, an acyl coenzyme A -cholesterol acyl transferase (ACAT) inhibitor, a squalene synthetase inhibitor, an anti-oxidant, a FXR receptor modulator, a LXR receptor agonist, a lipoprotein synthesis inhibitor, a microsomal triglyceride transport inhibitor, a bile acid reabsorption inhibitor, a triglyceride synthesis inhibitor, a transcription modulator, a squalene epoxidase inhibitor, a low density lipoprotein (LDL) receptor inducer, a 5-LO or FLAP inhibitor, and niacin or a niacin receptor agonist, or a pharmaceutically acceptable salt thereof; and
(b) an anti-obesity agent selected from the group consisting of a 5HT (serotonin) transporter inhibitor, a NE (norepinephrine) transporter inhibitor, a ghrelin antibody, a ghrelin antagonist, a H3 (histamine H3) antagonist/inverse agonist, a MCH1 R (melanin concentrating hormone 1 R) antagonist, a MCH2R (melanin concentrating hormone 2R) agonist/antagonist, a NPY1 (neuropeptide Y Y1) antagonist, a NPY2 (neuropeptide Y Y2) agonist, a NPY5 (neuropeptide Y Y5) antagonist, leptin, a leptin derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a GHS (growth hormone secretagogue receptor) agonist, 5HT2c (serotonin receptor 2c) agonist, a Mc3r (melanocortin 3 receptor) agonist, a Mc4r (melanocortin 4 receptor) agonist, a monoamine reuptake inhibitor, a serotonin reuptake inhibitor, topiramate, phytopharm compound 57, an ACC2 (acetyl-CoA carboxylase-2) inhibitor, a β3 (beta adrenergic receptor 3) agonist, a FAS (fatty acid synthase) inhibitor, a PDE (phosphodiesterase) inhibitor, a thyroid hormone,B agonist, an UCP-1 (uncoupling protein 1), 2, or 3 activator, an acyl-estrogen, a glucocorticoid antagonist, an 110 HSD-1 (11-beta hydroxy steroid debydrogenase type 1) inhibitor, a lipase inhibitor, a fatty acid transporter inhibitor, a dicarboxylate transporter inhibitor, a glucose transporter inhibitor, and a phosphate transporter inhibitor,
   or a pharmaceutically acceptable salt thereof.

Another example of an anti-dyslipidemic agent mentioned abovew under (a) is a cholesteryl ester transfer protein inhibitor. Consequently, the present invention is also related to a combination of at least two components selected from the group consisting of:
(i) a renin inhibitor or a renin inhibitor combined with a diuretic a pharmaceutically acceptable salt thereof; and
(ii) a cholesteryl ester transfer protein inhibitor or a pharmaceutically acceptable salt thereof.

Furthermore, the present invention provides pharmaceutical compositions comprising a renin inhibitor, or a pharmaceutically acceptable salt thereof, and at least one therapeutic agent selected from the group consisting of
(a) an anti-dyslipidemic agent selected from the group consisting of a bile acid sequesterant, a cholesterol absorption inhibitor, an acyl coenzyme A -cholesterol acyl transferase (ACAT) inhibitor, a squalene synthetase inhibitor, an anti-oxidant, a FXR receptor modulator, a LXR receptor agonist, a lipoprotein synthesis inhibitor, a microsomal triglyceride transport inhibitor, a bile acid reabsorption inhibitor, a triglyceride synthesis inhibitor, a transcription modulator, a squalene epoxidase inhibitor, a low density lipoprotein (LDL) receptor inducer, a 5-LO or FLAP inhibitor, and niacin or a niacin receptor agonist,
   or a pharmaceutically acceptable salt thereof; and
(b) an anti-obesity agent selected from the group consisting of a 5HT (serotonin) transporter inhibitor, a NE (norepinephrine) transporter inhibitor, a ghrelin antibody, a ghrelin antagonist, a H3 (histamine H3) antagonist/inverse agonist, a MCH1R (melanin concentrating hormone 1 R) antagonist, a MCH2R (melanin concentrating hormone 2R) agonist/antagonist, a NPY1 (neuropeptide Y Y1) antagonist, a NPY2 (neuropeptide Y Y2) agonist, a NPY5 (neuropeptide Y Y5) antagonist, leptin, a leptin derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a GHS (growth hormone secretagogue receptor) agonist, 5HT2c (serotonin receptor 2c) agonist, a Mc3r (melanocortin 3 receptor) agonist, a Mc4r (melanocortin 4 receptor) agonist, a monoamine reuptake inhibitor, a serotonin reuptake inhibitor, topiramate, phytopharm compound 57, an ACC2 (acetyl-CoA carboxylase-2) inhibitor, a β3 (beta adrenergic receptor 3) agonist, a FAS (fatty acid synthase) inhibitor, a PDE (phosphodiesterase) inhibitor, a thyroid hormone,B agonist, an UCP-1 (uncoupling protein 1), 2, or 3 activator, an acyl-estrogen, a glucocorticoid antagonist, an 110 HSD-1 (11-beta hydroxy steroid debydrogenase type 1) inhibitor, a lipase inhibitor, a fatty acid transporter inhibitor, a dicarboxylate transporter inhibitor, a glucose transporter inhibitor, and a phosphate transporter inhibitor,
   or a pharmaceutically acceptable salt thereof;
   and a pharmaceutically acceptable carrier.

Furthermore, the present invention also provides pharmaceutical compositions comprising a renin inhibitor, or a pharmaceutically acceptable salt thereof, and a cholesteryl ester transfer protein inhibitor or a pharmaceutically acceptable salt thereof.

Furthermore, the present invention provides a method for the prevention of, delay the onset of or treatment of dyslipidemia, dyslipidemia associated with obesity, dyslipidemia-related disorders, obesity, obesity-related disorders and/or a disease or a condition modulated by the inhibition of renin activity, which method comprises administering to a warm-blooded animal, including man, in need thereof, a therapeutically effective amount of a combination comprising a renin inhibitor, or a pharmaceutically acceptable salt thereof, and at least one therapeutic agent selected from the group consisting of
(a) an anti-dyslipidemic agent selected from the group consisting of a bile acid sequesterant, a cholesterol absorption inhibitor, an acyl coenzyme A -cholesterol acyl transferase (ACAT) inhibitor, a squalene synthetase inhibitor, an anti-oxidant, a FXR receptor modulator, a LXR receptor agonist, a lipoprotein synthesis inhibitor, a microsomal triglyceride transport inhibitor, a bile acid reabsorption inhibitor, a triglyceride synthesis inhibitor, a transcription modulator, a squalene epoxidase inhibitor, a low density lipoprotein (LDL) receptor inducer, a 5-LO or FLAP inhibitor, and niacin or a niacin receptor agonist,
   or a pharmaceutically acceptable salt thereof; and
(b) an anti-obesity agent selected from the group consisting of a 5HT (serotonin) transporter inhibitor, a NE (norepinephrine) transporter inhibitor, a ghrelin antibody, a ghrelin antagonist, a H3 (histamine H3) antagonist/inverse agonist, a MCH1 R (melanin concentrating hormone 1 R) antagonist, a MCH2R (melanin concentrating hormone 2R) agonist/antagonist, a NPY1 (neuropeptide Y Y1) antagonist, a NPY2 (neuropeptide Y Y2) agonist, a NPY5 (neuropeptide Y Y5) antagonist, leptin, a leptin derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a·GHS (growth hormone secretagogue receptor) agonist, 5HT2c (serotonin receptor 2c) agonist, a Mc3r (melanocortin 3 receptor) agonist, a Mc4r (melanocortin 4 receptor) agonist, a monoamine reuptake inhibitor, a serotonin reuptake inhibitor, topiramate, phytopharm compound 57, an ACC2 (acetyl-CoA carboxylase-2) inhibitor, a β3 (beta adrenergic receptor 3) agonist, a FAS (fatty acid synthase) inhibitor, a PDE (phosphodiesterase) inhibitor, a thyroid hormone,B agonist, an UCP-1 (uncoupling protein 1), 2, or 3 activator, an acyl-estrogen, a glucocorticoid antagonist, an 110 HSD-1 (11-beta hydroxy steroid debydrogenase type 1) inhibitor, a lipase inhibitor, a fatty acid transporter inhibitor, a dicarboxylate transporter inhibitor, a glucose transporter inhibitor, and a phosphate transporter inhibitor,
   or a pharmaceutically acceptable salt thereof.

Furthermore, the present invention provides a method for the prevention of, delay the onset of or treatment of dyslipidemia, dyslipidemia associated with obesity, dyslipidemia-related disorders, obesity, obesity-related disorders and/or a disease or a condition modulated by the inhibition of renin activity, which method comprises administering to a warm-blooded animal, including man, in need thereof, a therapeutically effective amount of a combination comprising a renin inhibitor, or a pharmaceutically acceptable salt thereof, and a cholesteryl ester transfer protein inhibitor or a pharmaceutically acceptable salt thereof.

### Definitions

The term "at least one therapeutic agent" shall mean that in addition to a renin inhibitor one or more, for example, two, furthermore three, active ingredients as specified according to the present invention can be combined. This means in particular that beside the renin inhibitor one or more, e.g. two or three, anti-obesity agents can be present. Likewise, this means in particular that beside the renin inhibitor one or more, e.g. two or three, anti-dyslipidemic agents can be present. Furthermore, one or more of both the anti-dyslipidemic and the anti-obesity agents can be present.

The term "combination" of a renin inhibitor, or a pharmaceutically acceptable salt thereof, and at least one therapeutic agent selected from the group consisting of (a) and (b) as defined above, means that the components can be administered together as a pharmaceutical composition or as part of the same, unitary dosage form. A combination also includes administering a renin inhibitor, or a pharmaceutically acceptable salt thereof, and at least one therapeutic agent selected from the group consisting of (a) and (b) as defined above, each separately but as part of the same therapeutic regimen. The components, if administered separately, need not necessarily be administered at essentially the same time, although they can if so desired. Thus, a combination also refers, for example, administering a renin inhibitor, or a pharmaceutically acceptable salt thereof, and at least one therapeutic agent selected from the group consisting of (a) and (b) as defined above, as separate dosages or dosage forms, but at the same time. A combination also includes separate administration at different times and in any order.

The term "prevention" refers to prophylactic administration to healthy patients to prevent the development of the conditions mentioned herein. Moreover, the term "prevention" means prophylactic administration to patients being in a pre-stage of the conditions to be treated.

The term "delay the onset of', as used herein, refers to administration to patients being in a pre-stage of the condition to be treated in which patients with a pre-form of the corresponding condition is diagnosed.

The term "treatment" is understood the management and care of a patient for the purpose of combating the disease, condition or disorder.

The term "therapeutically effective amount" refers to an amount of a drug or a therapeutic agent that will elicit the desired biological or medical response of a tissue, system or an animal (including man) that is being sought by a researcher or clinician.

The term "warm-blooded animal or patient" are used interchangeably herein and include, but are not limited to, humans, dogs, cats, horses, pigs, cows, monkeys, rabbits, mice and laboratory animals. The preferred mammals are humans.

The term "pharmaceutically acceptable salt" refers to a non-toxic salt commonly used in the pharmaceutical industry which may be prepared according to methods well-known in the art.

Diseases or a conditions modulated by the inhibition of renin activity as defined in the present invention include, but are not limited to, hypertension, congestive heart failure, diabetes, especially type 2 diabetes mellitus, mature onset diabetes of the young (MODY), diabetic retinopathy, macular degeneration, diabetic nephropathy, hypertensive or non-hypertensive nephropathy, IgA nephropathy, glomerulosclerosis, chronic renal failure, diabetic neuropathy, metabolic syndrome, cardiac syndrome X, atherosclerosis, coronary heart disease, angina pectoris, myocardial infarction, stroke, coronary and vascular restenosis, hyperglycemia, hyperinsulinaemia, hyperlipidemia, hypertryglyceridemia, insulin resistance, impaired glucose metabolism (IGM), conditions of impaired glucose tolerance. (IGT), IGM and/or IGT or increased inflammation in women with polycystic ovary syndrome or women with prior gestational diabetes, conditions of impaired fasting plasma glucose, obesity, diabetic retinopathy, macular degeneration, cataracts, foot ulcerations, endothelial dysfunction, impaired vascular compliance and obstructive sleep apnea. Preferably, said combination may be used for the treatment of hypertension, including ISH, as well as congestive heart failure, metabolic syndrome, endothelial dysfunction, impaired vascular compliance, IGT, diabetes especially type II diabetes mellitus, hypertensive or non-hypertensive nephropathy, IgA nephropathy, as well as retardation or prolongation of the progression of prediabetes to diabetes. Most preferably, Preferably, said combination may be used for the treatment of hypertension, including ISH, as well as congestive heart failure, metabolic syndrome, diabetes especially type II diabetes mellitus, and hypertensive or non-hypertensive nephropathy.

The term "dyslipidemia" includes various disorders of lipid metabolism, such as conditions characterized by abnormal concentrations of one or more lipids (i.e. cholesterol and triglycerides), and/or apolipoproteins (i.e., apolipoproteins A, B. C and E), and/or lipoproteins (i.e., the macromolecular complexes formed by the lipid and the apolipoprotein that allow lipids to circulate in blood, such as LDL,VLDL and IDL). The term "dyslipidemia" includes hyperlipidemia, which is associated with abnormally high levels of lipids, LDL and VLDL cholesterol, and/or triglycerides.

Dyslipidemia and dyslipidemia-related disorders as defined in the present invention may be due to any cause, whether genetic or environmental. The compositions of the present invention are useful for the treatment, control or prevention of dyslipidernia associated with obesity. The term "dyslipidemia associated with obesity" refers to dyslipidemia caused by obesity or resulting from obesity. Treatment of dyslipidemia, or a dyslipidemia related disorder, refers to the administration of the combinations of the present invention to a dyslipidemia subject. Prevention of dyslipidemia, or a dyslipidemia associated disorder, refers to the administration of the combinations of the present invention to a pre-dyslipidemic subject. A pre-dyslipidemic subject is a subject with higher than normal lipid levels that is not yet dyslipidemic. Thus, prevention of dyslipidemia refers to the administration of a compound or combination of the present invention to prevent the onset of dyslipidemia in a subject in need thereof.

The compositions of the present invention are also useful for the treatment, control, or prevention of; dyslipidemia-related disorders. The dyslipidemia-related disorders herein are associated with, caused by, or result from dyslipidemia. Examples of dyslipidemia-related disorders include lipid disorders, hyperlipidemia, hypertriglyceridemia, hypercholesterolemia, low high density lipoprotein (HDL) levels, high plasma low density lipoprotein (LDL) levels, atherosclerosis and its sequelae, coronary artery or carotid artery disease, heart attack, and stroke. In particular, dyslipidemia-related disorders include hyperlipidemia, hypertriglyceridemia, hypercholesterolernia, low high density lipoprotein (HDL) levels, high plasma low density lipoprotein (LDL) levels, and atherosclerosis. Dyslipidemia-related disorders further include metabolic syndrome.

The compositions of the present invention are expected to be efficacious in the treatment of lipid disorders, such as dyslipidemia, hyperlipidemia, hypercholesterolemia, hypertriglyceridemia, and low levels of high density lipoproteins; for lowering plasma low density lipoprotein and/or total cholesterol, for lowering plasma triglycerides, for preventing or reducing the risk of developing atherosclerosis, as well as for halting or slowing the progression of atherosclerotic disease once it has become clinically evident. The compositions of the present invention are also useful in the treatment, control and/or prevention of coronary artery or carotid artery disease, heart attack, and stroke. The term dyslipidemia and dyslipidemia-related disorder thus includes hyperlipidemia, hypertriglyceridemia, hypercholesterolemia, low high density lipoprotein cholesterol levels, high low density lipoprotein cholesterol levels, atherosclerosis, coronary artery disease, heart attack, and stroke.

The compositions of the present invention are useful for the treatment, control, or prevention of cardiac hypertrophy and/or left ventricular hypertrophy.

The compositions of the present invention are also useful for the treatment, control, or prevention of metabolic syndrome. The compositions of the present invention are useful for the treatment, control, or prevention of obesity and obesity-related disorders. The obesity herein may be due to any cause, whether genetic or environmental.

The term "obesity" as used herein is a condition in which there is an excess of body fat. The operational definition of obesity is based on the Body Mass Index (BMI), which is calculated as body weight per height in meters squared (kg/m2). "Obesity" refers to a condition whereby an otherwise healthy subject has a Body Mass Index (BMI) greater than or equal to 30 kg/m2, or a condition whereby a subject with at least one co- morbidity has a BMI greater than or equal to 27 kg/m2. An "obese subject" is an otherwise healthy subject with a Body Mass Index (BMI) greater than or equal to 30 kg/m2 or a subject with at least one co-morbidity with a BMI greater than or equal to 27 kg/m2. A "subject at risk of obesity" is an otherwise healthy subject with a BMI of 25 kg/m2 to less than 30 kg/m2 or a subject with at least one co-morbidity with a BMI of 25 kg/m2 to less than 27 kg/m2. The increased risks associated with obesity occur at a lower Body Mass Index (BMI) in Asians. In Asian countries, including Japan, "obesity" refers to a condition whereby a subject with at least one lo obesity-induced or obesity-related co-morbidity, that requires weight reduction or that would be improved by weight reduction, has a BMI greater than or equal to 25 kg/m2. In Asian countries, including Japan, an "obese subject" refers to a subject with at least one obesity-induced or obesity- related co-morbidity that requires weight reduction or that would be improved by weight reduction, with a BMI greater than or equal to 25 kg/m2. In Asia-Pacifc, a "subject at risk of obesity" is a subject with a 15 BMI of greater than 23 kg/m2 to less than 25 kg/m2.

As used herein, the term "obesity" is meant to encompass all of the above definitions of obesity.

Obesity-induced or obesity-related co-morbidities include, but are not limited to, diabetes, non- insulin dependent diabetes mellitus - type 2, diabetes associated with obesity, impaired glucose tolerance, impaired fasting glucose, insulin resistance syndrome, dyslipidemia, hypertension, hypertension associated with obesity, hyperuricemia, gout, coronary artery disease, myocardial infarction, angina pectoris, sleep apnea syndrome, Pickwickian syndrome, fatty liver; cerebral infarction, cerebral thrombosis, transient ischemic attack, orthopedic disorders, arthritis deformans, lumbodynia, emmeniopathy, and infertility. In particular, co-morbidities include: hypertension, hyperlipidemia, dyslipidemia, glucose intolerance, cardiovascular disease, sleep apnea, diabetes mellitus, and other obesity-related conditions.

Treatment of obesity and obesity-related disorders refers to the administration of the compounds or combinations of the present invention to reduce or maintain the body weight of an obese subject. One outcome of treatment may be reducing the body weight of an obese subject relative to that subject's body; weight immediately before the administration of the compounds or combinations of the present invention. Another outcome of treatment may be preventing body weight regain of body weight previously lost as a result of diet, exercise, or pharmacotherapy. Another outcome of treatment may be decreasing the occurrence of and/or the severity of obesity-related diseases. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

Prevention of obesity and obesity-related disorders refers to the administration of the compounds or combinations of the present invention to reduce or maintain the body weight of a subject at risk of obesity. One outcome of prevention may be reducing the body weight of a subject at risk of obesity relative to that subject's body weight immediately before the administration of the compounds or combinations of the present invention. Another outcome of prevention may be preventing body weight regain of body weight previously lost as a result of diet, exercise, or pharmacotherapy. Another outcome of prevention may be preventing obesity from occurring if the treatment is administered prior to the onset of obesity in a subject at risk of obesity. Another outcome of prevention may be decreasing the occurrence and/or severity of obesity-related disorders if the treatment is administered prior to the onset of obesity in a subject at risk of obesity. Moreover, if treatment is commenced in already obese subjects, such treatment may prevent the occurrence, progression or severity of obesity-related disorders, such as, but not limited to, arteriosclerosis, Type 2 diabetes, polycystic ovary disease, cardiovascular diseases, osteoarthritis, dermatological disorders, hypertension, insulin resistance, hypercholesterolemia, hypertriglyceridemia, and cholelithiasis.

The obesity-related disorders herein are associated with, caused by, or result from obesity. Examples of obesity-related disorders include diabetes, overeating, binge eating, and bulimia, hypertension, elevated plasma insulin concentrations and insulin resistance, dyslipidemias, hyperlipidemia, endometrial, breast, prostate, kidney and colon cancer, osteoarthritis, obstructive sleep apnea, gallstones, heart disease, abnormal heart rhythms and arrythmias, myocardial infarction, congestive heart failure, coronary heart disease, sudden death, stroke, polycystic ovary disease, craniopharyngioma, the Prader-Willi Syndrome, Frohlich's syndrome, GH- deficient subjects, normal variant short stature, Turner's syndrome, and other pathological conditions showing reduced metabolic activity or a decrease in resting energy expenditure as a percentage of total fat-free mass, e.g. children with acute lymphoblastic leukemia. Further examples of obesity-related disorders are metabolic syndrome, also known as syndrome X, insulin resistance syndrome, reproductive hormone abnormalities, sexual and reproductive dysfunction, such as impaired fertility, infertility, hypogonadism in males and: hirsutism in females, fetal defects associated with maternal obesity, gastrointestinal motility disorders, such as obesity-related gastro-esophageal reflex, respiratory disorders, such as obesity-hypoventilation syndrome (Pickwickian syndrome), breathlessness, cardiovascular disorders, inflammation, such as systemic inflammation of the vasculature, arteriosclerosis, hypercholesterolemia, hyperuricaemia, lower back pain, gallbladder disease, hyperuricemia, gout, and kidney cancer, and increased anesthetic risk. The compositions of the present invention are also useful to treat Alzheimer's disease.

The term "atherosclerosis" as used herein encompasses vascular diseases and conditions that are recognized and understood by physicians practicing in the relevant fields of medicine. Atherosclerotic cardiovascular disease, coronary heart disease (also known as coronary artery disease or ischemic heart disease), cerebrovascular disease and peripheral vessel disease are all clinical manifestations of atherosclerosis and are therefore encompassed by the terms "atherosclerosis" and "atherosclerotic disease." The combination comprised of a therapeutically effective amount of an anti-obesity agent in combination with a therapeutically effective amount of an anti- dyslipidemic agent may be administered to prevent or reduce the risk of occurrence, or recurrence where the potential exists, of a coronary heart disease event, a cerebrovascular event, or intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events, and intermittent claudication. It is intended that persons who have previously experienced one or more non-fatal atherosclerotic disease events are those for whom the potential for recurrence of such an event exists.

The term "metabolic syndrome", also known as syndrome X, is defined in the Third Report of the National Cholesterol Education Program Expert Panel on Detection, Evaluation and Treatment of High Blood Cholesterol in Adults (ATP-III). E.S. Ford et al., JAMA, vol. 287 (3), Jan. 16, 2002, pp 356 359. Briefly, a person is defined as having metabolic syndrome if the person has three or more of the following disorders: abdominal obesity, hypertriglyceridemia, low HDL cholesterol, high blood pressure, and high fasting plasma glucose. The criteria for these are defined in ATP-111. Treatment of metabolic syndrome refers to the administration of the combinations of the present invention to a subject with metabolic syndrome. Prevention of metabolic syndrome refers to the administration of the combinations of the present invention to a subject with two of the disorders that define metabolic syndrome. A subject with two of the disorders that define metabolic syndrome is a subject that has developed two of the disorders that define metabolic syndrome, but has not yet developed three or more of the disorders that define metabolic syndrome.

Left ventricular hypertrohpy (LVH) is identified based on left ventricular mass index (LVMI) and relative wall thickness (RWT). Left ventricular mass index is defined as left ventricular mass in i grams divided by body surface area in meters. Relative wall thickness is defined as 2 x posterior wall I thickness/left ventricular end diastolic diameter. Normal LVMI values are typically 85 and normal RWT approximately 0.36. A male subject with LVH has a LVMI greater than 131 g/m2; a female subject with LVH has a LVMI greater than 100 g/m2. A subject with an elevated LVMI value is a male subject with a LVMI between 85 g/m2 and 131 g/m2, or a female subject with a LVMI between 85 g/m2 and 100 g/m2. Treatment of cardiac hypertrophy, or left ventricular hypertrophy, refers to the administration of the combinations of the present invention to a subject with cardiac hypertrophy or left ventricular hypertrophy. Prevention of cardiac hypertrophy, or left ventricular hypertrophy, refers to the administration of the combinations of the present invention to decrease or maintain the LVMI in a subject with an elevated LVMI value or to prevent the increase of LVMI in a subject with a normal LVMI value. One outcome of treatment of cardiac hypertrophy or left ventricular hypertrophy may be a decrease in ventricular mass. Another outcome of treatment of cardiac hypertrophy or left ventricular hypertrophy may be a decrease in the rate of increase of ventricular mass. Another outcome of treatment of cardiac hypertrophy or left ventricular hypertrophy may be a decrease in ventricular wall thickness.

Another outcome of treatment of cardiac hypertrophy of left ventricular hypertrophy may be the decrease in the rate of increase in ventricular wall thickness.

### Renin inhibitors

The natural enzyme renin released from the kidneys cleaves angiotensinogen in the circulation to form the decapeptide called angiotensin I. This in turn is cleaved by angiotensin converting enzyme (ACE) in the lungs, kidneys and other organs to form the octapeptide called angiotensin II. Through its interaction with specific receptors on the surface of the target cells the octapeptide increases blood pressure both directly by arterial vasoconstriction and indirectly by liberating from the adrenal glands the sodium-ion-retaining hormone aldosterone, accompanied by an increase in extracellular fluid volume. It has been possible to identify receptor subtypes that are termed, e.g., AT₁- and AT₂-receptors. Inhibitors of the enzymatic activity of renin bring about a reduction in the formation of angiotensin I. As a result a smaller amount of angiotensin II is produced. The reduced concentration of that active peptide hormone is the direct cause of, e.g., the antihypertensive effect of renin inhibitors. Accordingly, renin inhibitors, or salts thereof, may be employed, e.g., as antihypertensives or for treating congestive heart failure.

The renin inhibitors to which the present invention applies are any of those having renin inhibitory activity *in vivo* and, therefore, pharmaceutical utility, e.g., as therapeutic agents for the prevention of, delay the onset of or treatment of hypertension, congestive heart failure, diabetes, especially type 2 diabetes mellitus, diabetic retinopathy, macular degeneration, diabetic nephropathy, hypertensive or non-hypertensive nephropathy and IgA nephropathy, glomerulosclerosis, renal failure, especially chronic renal failure, diabetic neuropathy, metabolic syndrome, cardiac syndrome X, atherosclerosis, coronary heart disease, angina pectoris, myocardial infarction, stroke, coronary and vascular restenosis, endothelial dysfunction, arterial stiffness, and the like.

In particular, the present invention relates to renin inhibitors disclosed in U.S. Patents No. 5,559,111 and EP 678503 A; No. 6,197,959 and No. 6,376,672, the entire contents of which are incorporated herein by reference.

Suitable renin inhibitors include compounds having different structural features. For example, mention may be made of compounds which are selected from the group consisting of ditekiren (chemical name: [1S-[1R*,2R*,4R*(1R*,2R*)]]-1-[(1,1-dimethylethoxy)carbonyl]-L-proly I-L-phenylalanyl-N-[2-hydroxy-5-methyl-1-(2-methylpropyl)-4-[[[2-methyl-1-[[(2-pyridinylmrthyl)amino]carbonyl]butyl]amino]carbonyl]hexyl]-N-alfa-methyl-L-histidinamide); terlakiren (chemical name: [R-(R*,S*)]-N-(4-morpholinylcarbonyl)-L-phenylalanyl-N-[1-(cyclohexy Imethyl)-2-hydroxy-3-(1-methylethoxy)-3-oxopropyl]-S-methyl-L-cysteineamide); and zankiren (chemical name: [1S-[1R*[R*(R*)],2S*,3R*]]-N-[1-(cyclohexylmethyl)-2,3-dihydroxy-5-methylhexyl]-alfa-[[2-[[(4-methyl-1-piperazinyl)sulfonyl]methyl]-1-oxo-3-phenylpropyl]-amino]-4-thiazolepropanamide), preferably, in each case, the hydrochloride salt thereof.

Preferred renin inhibitor of the present invention include RO 66-1132 and RO 66-1168 of formulae (I) and (II) 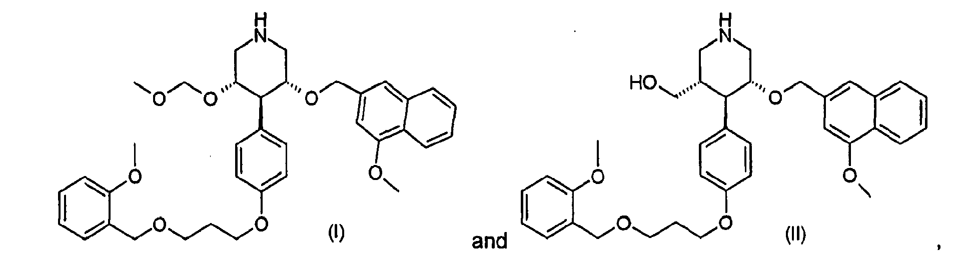 respectively, or a pharmaceutically acceptable salt thereof.

In particular, the present invention relates to a renin inhibitor which is is a δ-amino-γ-hydroxy-ω-aryl-alkanoic acid amide derivative of the formula 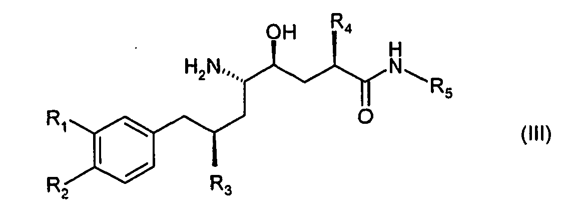 wherein R₁ is halogen, C₁₋₆halogenalkyl, C₁₋₆alkoxy-C₁₋₆alkyloxy or C₁₋₆alkoxy-C₁₋₆alkyl; R₂ is halogen, C₁₋₄alkyl or C₁-₄alkoxy; R₃ and R₄ are independently branched C₃-₆alkyl; and R₅ is cycloalkyl, C₁₋₆alkyl, C₁₋₆hydroxyalkyl, C₁₋₆alkoxy-C₁₋₆alkyl, C₁₋₆alkanoyloxy-C₁₋₆alkyl, C₁₋₆eaminoalkyl, C₁₋₆alkylamino-C₁₋₆alkyl, C₁₋₆dialkylamino-C₁₋₆alkyl, C₁₋₆alkanoylamino-C₁₋₆alkyl, HO(O)C-C₁₋₆alkyl, C₁₋₆alkyl-O-(O)C-C₁₋₆alkyl, H₂N-C(O)-C₁₋₆alkyl, C₁₋₆alkyl-HN-C(O)-C₁₋₆alkyl or (C₁₋₆alkyl)₂N-C(O)-C₁₋₆alkyl; or a pharmaceutically acceptable salt thereof.

As an alkyl, R₁ may be linear or branched and preferably comprise 1 to 6C atoms, especially 1 or 4C atoms. Examples are methyl, ethyl, n- and i-propyl, n-, i- and t-butyl, pentyl and hexyl.

As a halogenalkyl, R₁ may be linear or branched and preferably comprise 1 to 4 C atoms, especially 1 or 2 C atoms. Examples are fluoromethyl, difluoromethyl, trifluoromethyl, chloromethyl, dichloromethyl, trichloromethyl, 2-chloroethyl and 2,2,2-trifluoroethyl.

As an alkoxy, R₁ and R₂ may be linear or branched and preferably comprise 1 to 4 C atoms. Examples are methoxy, ethoxy, n- and i-propyloxy, n-, i- and t-butyloxy, pentyloxy and hexyloxy.

As an alkoxyalkyl, R₁ may be linear or branched. The alkoxy group preferably comprises 1 to 4 and especially 1 or 2 C atoms, and the alkyl group preferably comprises 1 to 4 C atoms. Examples are methoxymethyl, 2-methoxyethyl, 3-methoxypropyl, 4-methoxybutyl, 5-methoxypentyl, 6-methoxyhexyl, ethoxymethyl, 2ethoxyethyl, 3-ethoxypropyl, 4-ethoxybutyl, 5-ethoxypentyl, 6-ethoxyhexyl, propyloxymethyl, butyloxymethyl, 2-propyloxyethyl and 2-butyloxyethyl.

As a C₁₋₆alkoxy-C₁₋₆alkyloxy, R₁ may be linear or branched. The alkoxy group preferably comprises 1 to 4 and especially 1 or 2 C atoms, and the alkyloxy group preferably comprises 1 to 4 C atoms. Examples are methoxymethyloxy, 2-methoxyethyloxy, 3-methoxypropyloxy, 4-methoxybutyloxy, 5-methoxypentyloxy, 6-methoxyhexyloxy, ethoxymethyloxy, 2-ethoxyethyloxy, 3-ethoxypropyloxy, 4-ethoxybutyloxy, 5-ethoxypentyloxy, 6-ethoxyhexyloxy, propyloxymethyloxy, butyloxymethyloxy, 2-propyloxyethyloxy and 2-butyloxyethyloxy.

In a preferred embodiment, R₁ is methoxy- or ethoxy-C₁₋₄alkyloxy, and R₂ is preferably methoxy or ethoxy. Particularly preferred are compounds of formula (III), wherein R₁ is 3-methoxypropyloxy and R₂ is methoxy.

As a branched alkyl, R₃ and R₄ preferably comprise 3 to 6 C atoms. Examples are i-propyl, i-and t-butyl, and branched isomers of pentyl and hexyl. In a preferred embodiment, R₃ and R₄ in compounds of formula (III) are in each case i-propyl.

As a cycloalkyl, R₅ may preferably comprise 3 to 8 ring-carbon atoms, 3 or 5 being especially preferred. Some examples are cyclopropyl; cyclobutyl, cyclopentyl, cyclohexyl and cyclooctyl. The cycloalkyl may optionally be substituted by one or more substituents, such as alkyl, halo, oxo, hydroxy, alkoxy, amino, alkylamino, dialkylamino, thiol, alkylthio, nitro, cyano, heterocyclyl and the like.

As an alkyl, R₅ may be linear or branched in the form of alkyl and preferably comprise 1 to 6 C atoms. Examples of alkyl are listed herein above. Methyl, ethyl, n- and i-propyl, n-, i- and t-butyl are preferred.

As a C₁₋₆hydroxyalkyl, R₅ may be linear or branched and preferably comprise 2 to 6 C atoms. Some examples are 2-hydroxyethyl, 2-hydroxypropyl, 3-hydroxypropyl, 2-, 3- or 4-hydroxybutyl, hydroxypentyl and hydroxyhexyl.

As a C₁₋₆alkoxy-C₁₋₆aalkyl, R₅ may be linear or branched. The alkoxy group preferably comprises 1 to 4 C atoms and the alkyl group preferably 2 to 4 C atoms. Some examples are 2-methoxyethyl, 2-methoxypropyl, 3-methoxypropyl, 2-, 3- or 4-methoxybutyl, 2-ethoxyethyl, 2-ethoxypropyl, 3-ethoxypropyl, and 2-, 3- or 4-ethoxybutyl.

As a C₁₋₆alkanoyloxy-C₁₋₆alkyl, R₅ may be linear or branched. The alkanoyloxy group preferably comprises 1 to 4 C atoms and the alkyl group preferably 2 to 4 C atoms. Some examples are formyloxymethyl, formyloxyethyl, acetyloxyethyl, propionyloxyethyl and butyroyloxyethyl.

As a C₁₋₆aminoalkyl, R₅ may be linear or branched and preferably comprise 2 to 4 C atoms. Some examples are 2-aminoethyl, 2- or 3-aminopropyl and 2-, 3- or 4-aminobutyl.

As C₁₋₆alkylamino-C₁₋₆alkyl and C₁₋₆dialkylamino-C₁₋₆alkyl, R₅ may be linear or branched. The alkylamino group preferably comprises C₁₋₄alkyl groups and the alkyl group has preferably 2 to 4 C atoms. Some examples are 2-methylaminoethyl, 2-dimethylaminoethyl, 2-ethylaminoethyl, 2-ethylaminoethyl, 3-methylaminopropyl, 3-dimethylaminopropyl, 4-methylaminobutyl and 4-dimethylaminobutyl.

As a HO(O)C-C₁₋₈alkyl, R₅ may be linear or branched and the alkyl group preferably comprises 2 to 4 C atoms. Some examples are carboxymethyl, carboxyethyl, carboxypropyl and carboxybutyl.

As a C₁₋₆alkyl-O-(O)C-C₁₋₆alkyl, R₅ may be linear or branched, and the alkyl groups preferably comprise independently of one another 1 to 4 C atoms. Some examples are methoxycarbonylmethyl, 2-methoxycarbonylethyl, 3-methoxycarbonylpropyl, 4-methoxy-carbonylbutyl, ethoxycarbonylmethyl, 2-ethoxycarbonylethyl, 3-ethoxycarbonylpropyl, and 4-ethoxycarbonylbutyl.

As a H₂N-C(O)-C₁₋₆alkyl, R₅ may be linear or branched, and the alkyl group preferably comprises 2 to 6 C atoms. Some Examples are carbamidomethyl, 2-carbamidoethyl, 2-carbamido-2,2-dimethylethyl, 2- or 3-carbamidopropyl, 2-, 3- or 4-carbamidobutyl, 3-carbamido-2-methylpropyl, 3-carbamido-1,2-dimethylpropyl, 3-carbamido-3-ethylpropyl, 3-carbamido-2,2-dimethylpropyl, 2-, 3-, 4- or 5-carbamidopentyl, 4-carbamido-3,3- or -2,2-dimethylbutyl. Preferably, R₅ is 2-carbamido-2,2-dimethylethyl.

Accordingly, preferred are δ-amino-y-hydroxy-ω-aryl-alkanoic acid amide derivatives of formula (III) having the formula 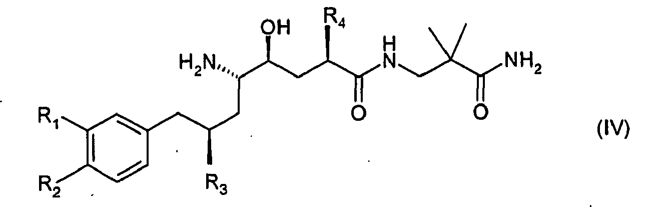 wherein R₁ is 3-methoxypropyloxy; R₂ is methoxy; and R₃ and R₄ are isopropyl; or a pharmaceutically acceptable salt thereof; chemically defined as 2(S),4(S),5(S),7(S)-N-(3-amino-2,2-dimethyl-3-oxopropyl)-2, 7-di(1-methylethyl)-4-hydroxy-5-amino-8-[4-methoxy-3-(3-methoxy-propoxy)phenyl]-octanamide, also known as aliskiren.

The term "aliskiren", if not defined specifically, is to be understood both as the free base and as a salt thereof, especially a pharmaceutically acceptable salt thereof, most preferably a hemi-fumarate salt thereof.

### Anti-dyslipidemic agents

The anti-dyslipidemic agents to which the present application applies can be readily identified by one skilled in the art or are identified in the Physician's Desk reference, Edition 56 (2002). Anti-dyslipidemic agents can be evaluated in a guinea pig model according to the procedures described in: Sullivan, M. et al., Lab. Anim. Sci. 43:575 778 (1993); Aoki, T., et al., Arzneimittelforschung 51:197-203, (2001). The term anti-dyslipidemic agent also includes within its meaning the compounds within the following therapeutic classes, or a combination of any of these compounds:

Bile acid sequestrants useful in the present invention include, but are not limited to, cholestyramine, colesevelem, colestipol (Colestid), dialkylaminoalkyl derivatives of a crosslinked dextran; LoCholest; and Questran. Thus, in one embodiment, the anti-dyslipidemic agent is a bile acid sequestrant. In a subclass of this class, the cholesterol I absorption inhibitor is cholestyramine; and pharmaceutically acceptable salts and esters thereof.

Cholesterol absorption inhibitors useful in the present invention include, but are not limited to, stanol esters, beta-sitosterol; sterol glycosides such as tiqueside; and azetidinones such as ezetimibe; and those disclosed in US 5,846,966, US 5,631,365, US 5,767,115, US 6,133,001, US 5,886,171, US 5,856,473, US 5,756,470, US 5,739,321, US 5,919,672, WO 00/63703, WO /0060107, WO 00/38725, WO 00/34240, WO 00/20623, WO 97/45406, WO 97/16424, WO 97/16455, and WO 95/08532, the entire contents of which are hereby incorporated by reference. An exemplary cholesterol absorption inhibitor is ezetimibe, which is 1-(4-fluorophenyl)-3 (R)-[3(S)- hydroxy-3-(4-fluorophenyl)propyl]-4(S) (4-hydroxyphenyl)-2-azetidinone. Additional exemplary hydroxy-substituted azetidinone cholesterol absorption inhibitors are specifically described in U.S. Patent 5,767,115, column 39, lines 54-61 and column 40, lines 1-51 (hereby incorporated by reference), represented by the formula as defined in column 2, lines 20-63 (hereby incorporated by reference). Thus, in one embodiment, the anti-dyslipidemic agent is a cholesterol absorption inhibitor, and pharmaceutically acceptable salts or esters thereof. In a class of this embodiment, the cholesterol absorption inhibitor is selected from the group consisting of beta-sitosterol, ezetimibe, and tiqueside; and] pharmaceutically acceptable salts and esters thereof. In a subclass of this class, the cholesterol I absorption inhibitor is ezetimibe; and pharmaceutically acceptable salts and esters thereof. In another embodiment, the anti-dyslipidemic agent is a composition comprising a cholesterol, absorption inhibitor, and pharmaceutically acceptable salts or esters thereof; and a HMG-CoA reductase I inhibitor, and pharmaceutically acceptable salts or esters thereof. In a class of this embodiment, the composition comprises ezetimibe, and pharmaceutically acceptable salts and esters thereof; and simvastatin, and pharmaceutically acceptable salts and esters thereof. In another class of this embodiment, the anti-dyslipidemic agent is vytorin.

Acyl coenzyme A -cholesterol acyl transferase (ACAT) inhibitors useful in the present invention include, but are not limited to, avasimibe, eflucimibe, KY505, SMP 797, and the like; and those disclosed in U.S. Patent No. 5,510,379, and International Patent Application Nos. WO 96/26948 and WO 96/10559. Thus, In one embodiment, the anti-dyslipidemic agent is an acyl coenzyme A - cholesterol acyl transferase (ACAT) inhibitor, and pharmaceutically acceptable salts or esters thereof. In a class of this embodiment, the acyl coenzyme A - cholesterol acyl transferase (ACAT) inhibitor is selected from the group consisting of avasimibe, eflucimibe, KY505 and SMP 797; and pharmaceutically acceptable salts and esters thereof. Thus, in one embodiment, the anti-dyslipidemic agent is an Acyl coenzyme A -cholesterol acyl transferase (ACAT) inhibitor.

Squalene synthetase inhibitors useful in the present invention include, but are not limited to, those dislosed in Curr. Op. Ther. Patents, 861-4, (1993); EP Patent Application Nos. 0 567 026 A1; 0 645 378 A1; 0 645 377 A1; 0 611 749 A1; 0 705 607 A2; 0 701 725 A1; and PCT publication WO 96/09827. Thus, in one embodiment, the anti-dyslipidemic agent is a Squalene synthetase inhibitor.

Anti-oxidants useful in the present invention include, but are not limited to, probucol; and pharmaceutically acceptable salts and esters thereof. Thus, In one embodiment, the anti-dyslipidemic agent is an anti-oxidant, and pharmaceutically acceptable salts or esters thereof. In a class of this embodiment, the anti-oxidant is selected from the group consisting of probucol; and pharmaceutically acceptable salts and esters thereof.

FXR receptor antagonists useful in the present invention include, but are not limited to, GW 4064, SR 103912, and the like. Thus, in one embodiment, the anti-dyslipidemic agent is a FXR receptor antagonist.

LXR receptor agonists useful in the present invention include, but are not limited to GW 3965, T9013137, and XTC0179628, and the like. Thus, in one embodiment, the anti-dyslipidemic agent is a LXR receptor agonist.

Lipoprotein synthesis inhibitors useful in the present invention include, but are not limited to, niacin or nicotinic acid. Thus, In one embodiment, the anti-dyslipidemic agent is a lipoprotein synthesis inhibitor, and pharmaceutically acceptable salts or esters thereof. In a class of this embodiment, the lipoprotein synthesis inhibitor is selected from the group consisting of niacin or nicotinic acid; and pharmaceutically acceptable salts and esters thereof.

Bile acid reuptake inhibitors useful in the present invention include, but are not limited to, BARI 1453, SC435, PHA384640, S8921, AZD7706, and the like. Thus, in one embodiment, the anti-dyslipidemic agent is a bile acid reuptake inhibitor.

Microsomal triglyceride transport inhibitors useful in the present invention include, but are not limited to, inplitapide, LAB687, and CP346086, and the like. Thus, in one embodiment, the anti-dyslipidemic agent is a microsomal triglyceride transport inhibitor.

The term CETP inhibitor refers to compounds that inhibit the cholesteryl ester transfer protein (CETP) mediated transport of various cholesteryl esters and triglycerides from HDL to LDL and VLDL. Such CETP inhibition activity is readily determined by those skilled in the art according to standard assays (e.g., U.S. Pat. No. 6,140,343). A variety of CETP inhibitors will be known to those skilled in the art, for example, those disclosed in commonly assigned U.S. Pat. No. 6,140,343 and commonly assigned, allowed, U.S. application Ser. No. 09/391,152. U.S. Pat. No. 5, 512,548 discloses certain polypeptide derivatives having activity as CETP inhibitors, while certain CETP-inhibitory rosenonolactone derivatives and phosphate-containing analogs of cholesteryl ester are disclosed in J. Antibiot ., 49(8): 815-816 (1996), and Bioorg. Med. Chem. Lett .; 6:1951-1954 (1996), respectively.

The other lipid agent or lipid-modulating agent may be a cholesteryl transfer protein inhibitor such as Pfizer's CP-529,414 as well as those disclosed in WO/0038722 and in EP 818448 (Bayer) and EP 992496, and Pharmacia's SC-744 and SC-795 as well as CETi-1 and JTT-705. Also included are the compounds described in WO2005/095409 and W02005/095395, especially in the Examples.

Inhibition of cholesteryl ester transfer protein has been shown to effectively modify plasma HDL/LDL ratios, and is expected to check the progress and/or formation of certain cardiovascular diseases. CETP is a plasma protein that facilitates the movement of cholesteryl esters and triglycerides between the various lipoproteins in the blood (Tall, J. Lipid Res., 34, 1255-74 (1993)). The movement of cholesteryl ester from HDL to LDL by CETP has the effect of lowering HDL cholesterol. It therefore follows that inhibition of CETP should lead to elevation of plasma HDL cholesterol and lowering of plasma LDL cholesterol, thereby providing a therapeutically beneficial plasma lipid profile. Evidence of this effect is ' described in McCarthy, Medicinal Res. Revs., 13, 139-59 (1993). Further evidence of this effect is described in Sitori, Pharmac. Ther., 67, 443-47 (1995)). This phenomenon was first demonstrated by Swenson et al., (J. Biol. Chem., 264, 14318 (1989)) with the use of a monoclonal antibody that specifically inhibits CETP. In rabbits, the antibody caused an elevation of the plasma HDL cholesterol and a decrease in LDL cholesterol. Son et al. ( Biochim. Biophys. Acta, 795, 743-480 (1984)) describe proteins from human plasma that inhibit CETP. U.S. Pat. No. 5,519,001, herein incorporated by reference, issued to Kushwaha et al., describes a 36 amino acid peptide derived from baboon apo C-1 that inhibits CETP activity. Cho et al. (Biochim. Biophys. Acta 1391, 133-144 (1998)) describe a peptide from hog plasma that inhibits human CETP. Bonin et al. (J. Peptide Res., 51, 216-225 (1998)) disclose a decapeptide inhibitor of CETP. A depspeptide fungal metabolite is disclosed as a CETP inhibitor by Hedge et al. in Bioorg. Med. Chem. Lett., 8, 1277-80 (1998).

There have been several reports of non-peptidic compounds that act as CETP inhibitors. Barrett et al. (J. Am. Chem. Soc., 188, 7863-63 (1996)) describe cyclopropane-containing CETP inhibitors. Further cyclopropane-containing CETP inhibitors are described by Kuo et al. (J. Am. Chem. Soc., 117, 10629-34 (1995)). Pietzonka et al. (Bioorg. Med. Chem. Lett., 6, 1951-54 (1996)) describe phosphonate- containing analogs of cholesteryl ester as CETP inhibitors. Coval et al. (Bioorg. Med. Chem. Lett., 5, 605-610 (1995)) describe Wiedendiol- A and -B, and related sesquiterpene compounds as CETP inhibitors. Lee et al. (J. Antibiotics, 49, 693-96 (1996)) describe CETP inhibitors derived from an insect fungus. Busch et al. ( Lipids, 25, 216-220, (1990)) describe cholesteryl acetyl bromide as a CETP inhibitor. Morton and Zilversmit (J. Lipid Res., 35, 836-47 (1982)) describe that p- chloromercuriphenyl sulfonate, p-hydroxymercuribenzoate and ethyl mercurithiosalicylate inhibit CETP. Connolly et al. (Biochem. Biophys. Res. Comm., 223, 42-47 (1996)) describe other cysteine modification reagents as CETP inhibitors. Xia et al. describe 1,3,5-triazines as.CETP inhibitors (Bioorg. Med. Chem. Lett., 6, 919-22 (1996)). Bisgaier et al. (Lipids, 29, 811-8 (1994)) describe 4-phenyl-5-tridecyl-4H- 1,2,4-triazole-thiol as a CETP inhibitor. Additional triazole CETP inhibitors are described in U.S. patent application Ser. No. 09/153,360, herein incorporated by reference. Sikorski et al. disclosed further novel CETP inhibitors in PCT Patent Application No. WO 9914204.

Substituted 2-mercaptoaniline amide compounds can be used as CETP inhibitors and such therapeutic compounds are described by H. Shinkai et al. in PCT Patent Application No. WO 98/35937.

Some substituted heteroalkylamine compounds are known as CETP inhibitors. In European Patent Application No. 796846, Schmidt et al. describe 2-aryl-substituted pyridines as cholesterol ester transfer protein inhibitors useful as cardiovascular agents. One substituent at C 3 of the pyridine ring can be an hydroxyalkyl group. In European Patent Application No. 801060, Dow and Wright describe heterocyclic derivatives substituted with an aldehyde addition product of an alkylamine to afford 1-hydroxy-1-amines. These are reported to be β3-adrenergic receptor agonists useful for treating diabetes and other disorders. In Great Britain Patent Application No. 2305665, Fisher et al. disclose 3-agonist secondary amino alcohol substituted pyridine derivatives useful for treating several disorders including cholesterol levels and atherosclerotic diseases. In European Patent Application No. 818448 (herein incorporated by reference), Schmidt et al. describe tetrahydroquinoline derivatives as cholesterol ester transfer protein inhibitors. European Patent Application No. 818197, Schmek et al. describe pyridines with fused heterocycles as cholesterol ester transfer protein inhibitors. Brandes et al. in German Patent Application No. 19627430 describe bicyclic condensed pyridine derivatives as cholesterol ester transfer protein inhibitors. In PCT Patent Application No. WO 9839299, Muller-Gliemann et al. describe quinoline derivatives as cholesteryl ester transfer protein inhibitors.

Polycyclic compounds that are useful as CETP inhibitors are also disclosed by A. Oomura et al. in Japanese Patent No. 10287662. For example, therapeutic compounds having the structures C-1 and C-8 were prepared by culturing Penicillium spp. Cycloalkylpyridines useful as CETP inhibitors are disclosed by Schmidt et al. in European

Patent No. EP 818448. For example, the therapeutic compound having the structure C-9 is disclosed as being particularly effective as a CETP inhibitor.

Substituted tetrahydronaphthalene compounds useful as CETP inhibitors are described in PCT Patent Application No. WO 9914174. Specifically described in that disclosure as a useful CETP inhibitor is (8S)-3- cyclopentyl-1-(4-fluorophenyl)-2-[(S)-fluoro(4-trifluoromethylphenyl) methyl]-8-hydroxy-6-spirocclobutyl-5,6,7,8-tetrahydronaphthalene. Some 4-heteroaryl-tetrahydroquinolines useful as CETP inhibitors are described in PCT Patent Application No. WO 9914215. For example, that disclosure describes 3-(4-trifluoromethylbenzoyl)-5,6,7,8- tetrahydroquinolin-5-one as a useful CETP inhibitor. Compounds useful in the present invention encompass a wide range of therapeutic compounds. Some individual CETP inhibitor compounds useful in the present invention are separately described in the following individual patent applications, each of which is herein incorporated by reference.

U.S. Pat. No. 5,932,587 and U.S. Pat. No. 5,925,645.

Cholesteryl ester transfer protein (CETP) inhibitors are known to participate in metabolism of all the lipoproteins in vivo and play an important role in reverse the transfer system of cholesterol. This system prevents the accumulation of cholesterol in peripheral cells and functions as a protective mechanism against atherosclerosis.

Thus, in one embodiment, the anti-dyslipidemic agent is a CETP inhibitor. Preferred CETP inhibitors comprise the compound with code JTT 705 of formula 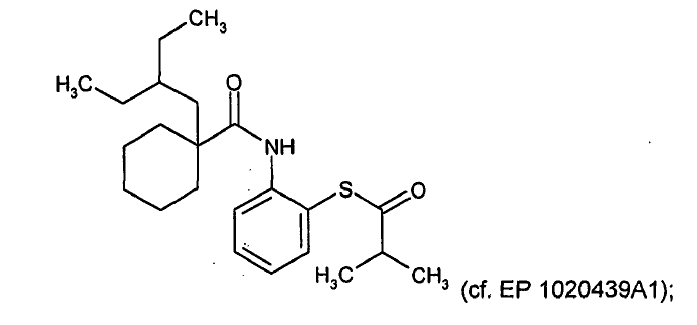 torcetrapib of formula

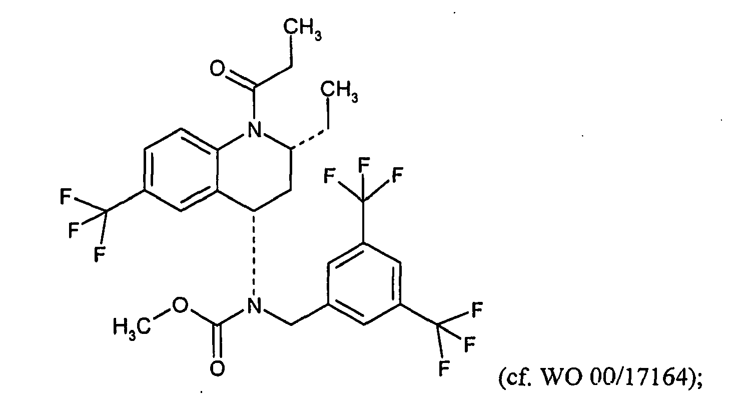

CP 532623 and the compounds described in W02005/095409 and WO2005/095395, especially in the Examples.

### Anti-Obesitv Agents

The Anti-Obesitv Agents to which the present application applies can be readily identified by one skilled in the art. Anti-obesity agents that are appetite suppressants can be evaluated in rodents according to the procedures described in: Daniels, A.J. et al., Regulatory Peptides, 106:47-54 - 32 (2002); Halaas, J.L. et. al., Science, 269: 5.43-546 (1995); and Strack, A.M., Obesity Research, 10:173-81 (2002). Anti-obesity agents that are metabolic rate enhancers are routinely evaluated in rodents (Atgie, C., Comp. Biochem. Physiol. A. Mol. lntegr. Physiol. 119:629-36 (1998); Himms-Hagan, J., American J. Physiology, 266:R1371-82 (1994)), and, even when inactive in rodents, are tested in additional species such as dog and monkey before ultimately being tested in humans (Connacher, A.A. et. al., Int'l J. Obesity, 16: 685-694 (1992); Connacher, A.A. et. al., Am. J. Clin. Nutr., 55: 258S- 261S (1992); Connacher, A.A. et. al., Brit. Med. J., 296: 1217-1220 (1998)). The utility of metabolic rate enhancers is supported by experiments with mice, in which the RII-beta gene has been deleted, that were shown to be resistant to diet induced obesity (D. E. Cummings et al. Nature 382: 622-626 (1996)). Anti-obesity agents that are nutrient absorption inhibitors can be evaluated in: Badr M.Z. and Chen, T.S., Toxicology, 34:333-40 (1985); Sorribas, V., J. Pharm. Pharmacol., 44:1030-2 (1992).

As used herein, the term "appetite suppressant" includes compounds that reduce total food intake by more than 5 %, or reduce caloric intake or selectively reduce intake of specific components of the diet such as carbohydrates or fats by more than 5 %. As used herein, the term "metabolic rate enhancer" includes compounds which, when administered to a subject, act to increase the metabolic rate of the subject, particularly those agents which increase metabolic rate by at least 5%, preferably 10%, most preferably 20% in 24 hour energy expenditure, and/or increase the oxidation of fatty acids relative to carbohydrates when administered to the subject. As used herein, the term "nutrient absorption inhibitor" includes compounds that inhibit the absorption of more than 10 % of the nutrients. Among the anti-obesity agents useful in the compositions of this invention are those identified, with dosing information, in the Physician's Desk reference, Edition 56 (2002). The term anti-obesity agent also includes within its meaning the compounds within the following therapeutic classes, or a combination of any of these compounds:

Serotonin (SHT) transport inhibitors useful in this invention include, but are not limited to, paroxetine, fluoxetine, fenfluramine, fluvoxamine, sertraline, and imipramine. Thus, in one embodiment, the anti-dyslipidemic agent is a Serotonin (SHT) transport inhibitor.

Norepinephrine (NE) transport inhibitors useful in this invention include, but are not limited to, GW 320659, despiramine, talsupram, and nomifensine. Thus, in one embodiment, the anti-dyslipidemic agent is a Norepinephrine (NE) transport inhibitor.

Ghrelin antagonists useful in the present invention, include those in US2003087821 and NZ 504256. Thus, in one embodiment, the anti-dyslipidemic agent is a Ghrelin antagonist.

Histamine 3 (H3) antagonist/inverse agonists useful in the present invention include: PCT Application No. WO 02/15905; and 0-[3-(IH-imidazol- 4-yl)propanol]carbamates (Kiec-Kononowicz, K. et al., Pharmazie, 55:349- 55 (2000)), piperidine-containing histamine H3-receptor antagonists (Lazewska, D. et al., Pharmazie, 56:927-32 (2001), benzophenone derivatives and related compounds (Sasse, A. et al., Arch. Pharm. (Weinheim) 334:45-52 (2001)), substituted N-phenylcarbamates (Reidemeister, S. et al., Pharmazie, 55:83-6 (2000)), and proxifan derivatives (Sasse, A. et al., J. Med. Chem.. 43:3335-43 (2000)). Specific H3 antagonists/inverse agonists useful in the present invention include, but are not limited to, thioperamide, 3-(IH-imidazol4-yl)propyl N-(4-pentenyl)carbamate, clobenpropit, iodophenpropit, imoproxifan, GT2394 (Gliatech), and A331440. Thus, in one embodiment, the anti-dyslipidemic agent is a Histamine 3 (H3) antagonist/inverse agonist.

Melanin-concentrating hormone 1 receptor (MCH1R) antagonists and melanin- concentrating hormone 2 receptor (MCH2R) agonist/antagonists useful in the present invention include PCT Patent Application Nos. WO 01/82925, WO 01/87834, we 02/06245, WO 02/04433, and WO 02/51809; and Japanese Patent Application No. JP 13226269. Specific MCH1 R antagonists useful in the present invention include, but are not limited to, T-226296 (Takeda), SB 568849, and SNAP 7941. Thus, in one embodiment, the anti-dyslipidemic agent is a Melanin-concentrating hormone 1 receptor (MCH1R) antagonist or melanin- concentrating hormone 2 receptor (MCH2R) agonist/antagonist.

Neuropeptide Y1 (NPY1) antagonists useful in the present invention, include: U.S. Patent No. 6,001,836; and PCT Application Nos. WO 96/14307, WO 01/23387, WO 99/51600, WO 01/85690, WO 01/85098, WO 01/85173, and WO 01/89528. Specific examples of NPY1 antagonists useful in the present invention include, but are not limited to, BIBP3226, J-115814, BIBO 3304, LY-357897, CP 671906, and GI-264879A. Thus, in one embodiment, the anti-dyslipidemic agent is a Neuropeptide Y1 (NPY1) antagonist.

Neuropeptide Y2 (NPY2) agonists useful in the present invention, include, but are not limited to, peptide YY (PYY), and PYY3 36, peptide YY analogs, PYY agonists, and the compounds disclosed in I WO 03/026591, WO 03/057235, and WO 03/027637. Thus, in one embodiment, the anti-obesity agent is a NPY2 agonist, and pharmaceutically acceptable salts or esters thereof. In a subclass of this class, the NPY2 agonist is selected from the group consisting of peptide YY (PYY), and PYY3 36, and pharmaceutically acceptable salts thereof. In another subclass of this class, the NPY2 agonist is PYY3 36, and pharmaceutically acceptable salts thereof. Neuropeptide Y2 (NPY2) agonist.

Neuropeptide Y5 (NPY5) antagonists useful in the present invention, include, but are not limited to, the compounds described in: U.S. Patent Nos. 6,140,354, 6,191,160, 6,258,837, 6,313,298, 6,337,332, 6,329,395, and 6,340,683; U.S. Patent Nos. 6,326,375; 6,329,395; 6,337,332; 6,335, 345; European Patent Nos. EP-01010691, and EP-01044970; and PCT International Patent Publication Nos. WO 97/19682, WO 97/20820, WO 97120821, WO 97/20822, WO 97/20823, WO 98/27063, WO 00/107409, WO 00/185714, WO 00/185730, WO 00/64880, WO 00/68197, WO 00/69849, WO 01/09120, WO 01/85714, WO 01/85730, WO 01 /07409, WO 01 /02379, WO 01/02379, WO 01 /23388, WO 01 /23389, WO 01 /44201, WO 01/62737, WO 01/62738, WO 01/09120, WO 02/20488, WO 02/22592, WO 02/48152, WO 02/49648, and WO 01/14376. Specific NPY 5 antagonists useful in the combinations of the present invention, include, but are not limited to GW-569180A, GW-594884A, GW-587081X, GW-548118X; FR 235,208; FR226928, FR 240662, FR252384; 1229U91, Gl-264879A, CGP71683A, LY-377897, LY366377, PD-160170, SR- 120562A, SR-120819A, JCF-104, and H409/22. Additional specific NPY 5 antagonists useful in the combinations of the present invention, include, but are not limited to the compounds described in Norman et al., J. Med. Chem. 43:42884312 (2000). Thus, in one embodiment, the anti-obesity agent is a NPY5 antagonist, and pharmaceutically acceptable salts or esters thereof. In a subclass of this class, the NPY5 antagonists useful in the present invention are represented by the compounds of structural Formula I as shown in WO2005/000217.

Leptin includes, but is not limited to, recombinant human leptin (PEG-OB, Hoffman La Roche) and recombinant methionyl human leptin (Amgen). Leptin derivatives (e.g., truncated forms of leptin) ; useful in the present invention include: Patent Nos. 5,552,524; 5,552,523; 5,552,522; 5,521,283; and PCT International Publication Nos. WO 96/23513; WO 96/23514; WO 96123515; WO 96/23516; WO 96/23517; WO 96/23518; WO 96/23519; and WO 96/23520. Thus, in one embodiment, the anti-dyslipidemic agent is a leptin.

Opioid antagonists useful in the present invention include: PCT Application No. WO; 00/21509. Specific opioid antagonists useful in the present invention include, but are not limited to, nalmefene (Revex), 3-methoxynaltrexone, naloxone, and naltrexone. In another class of this embodiment, the anti-obesity agent is an opioid antagonist, and pharmaceutically acceptable salts or esters thereof. Thus, in one embodiment, the opioid antagonist is selected from nalmefene, and pharmaceutically acceptable salts or esters thereof.

Orexin antagonists useful in the present invention include: PCT Patent Application Nos. WO 01/96302, WO 01/68609, WO 02/51232, WO 02/51838, and WO 03/023561. Specific orexin antagonists useful in the present invention include, but are not limited to, SB-334867-A. Thus, in one embodiment, the anti-dyslipidemic agent is a orexin antagonist.

Acyl-estrogens useful in the present invention include oleoyl-estrone (del Mar-Grasa, M. et al., Obesity Research, 9:202-9 (2001)). Thus, in one embodiment, the anti- obesity agent is an acyl-estrogen, and pharmaceutically acceptable salts or esters thereof. In a subclass of this class, the acyl-estrogen, is selected from oleoyl-estrone, and pharmaceutically acceptable salts or esters thereof.

Cholecystokinin-A (CCK-A) agonists useful in the present invention include U.S. Patent No. 5,739,106. Specific CCK-A agonists include, but are not limited to, AR-R 15849, GI 181771, JMV-180, A-71378, A-71623 and SR146131. CCK-A agonists useful in the present invention include GI 181771, and SR 146,131. Thus, in one embodiment, the anti-dyslipidemic agent is a Cholecystokinin-A (CCK-A) agonist.

Specific ciliary neurotrophic factors (CNTF) useful in the present invention include, but are not limited to, Gl-181771 (Glaxo-SrnithKline); SR146131 (Sanofi Synthelabo); butabindide; PD170,292, PD 149164 (Pfizer). CNTF derivatives useful in the present invention include, but are not limited to, axokine (Regeneron); and PCT Application Nos. WO 94/09134, WO 98/22128, and WO 99/43813. Thus, in one embodiment, the anti-obesity agent is a CNTF derivative, and pharmaceutically acceptable salts or esters thereof. In subclass of this class, the CNTF derivative is selected from axokine, and pharmaceutically acceptable salts or esters thereof.

Growth hormone secretagogue (OHS) agonists useful in the present invention include: U.S. Patent No. 6358951, and U.S. Patent Application Nos. 2002/049196 and 2002/022637; and PCT Application Nos. WO 01/56592, and WO 02/32888. Specific GHS agonists include, but are not limited to, NN703, hexarelin, MK-0677, SM-130686, CP- 424,391, L-692,429 and L-163,255. Thus, in one embodiment, the anti-dyslipidemic agent is a OHS agonist.

5HT2c agonists useful in the present invention include: U.S. Patent No. 3, 914,250; and PCT Application Nos. WO 02/36596, WO 02/48124, WO 02/10169, WO 01/66548, WO 02/44152; WO 02/51844, WO 02/40456, and WO 02/40457. Specific SHT2c agonists useful in this invention include, but are not limited to, BVT933, DPCA37215, IK264; PNU 22394; WAY161503, R-1065, and YM 348. Thus, in one embodiment, the anti-dyslipidemic agent is a 5HT2c agonist.

Mc4r agonists useful in the present invention include: PCT Application Nos. WO 99/64002, WO 00/74679, WO 01 /991752, WO 01 /74844, WO 01 /70708, WO 01 /70337, WO 01 /91752, WO 02/059095, WO 02/059 107, WO 02/059 108, WO 02/0591 17, WO 02/12166, WO 02/1 1715, WO 02/12178, WO 02/15909, WO 02/068387, WO 02/068388, WO 02/067869, WO 03/007949, and WO 03/009847. Specific Mc4r agonists useful in the present invention include CHIR86036 (Chiron); ME-10142, and ME-10145 (Melacure). Thus, in one embodiment, the anti-dyslipidemic agent is a Mc4r agonist.

Monoamine reuptake inhibitors useful in the present invention include: PCT Application Nos. WO 01/27068, and WO 01/62341. Specific monoamine reuptake inhibitors useful in the present invention include, but are not limited to, sibutramine (Meridia ()/Reductil) disclosed in U.S. Patent Nos. 4,746,680, 4,806,570, and 5,436,272, and U.S. Patent Publication No. 2002/0006964. The present; invention encompasses sibutratmine as a racemic mixture, as optically pure isomers (+) and (-), or a pharmaceutically acceptable salt, solvent, hydrate, clathrate or prodrug thereof; particularly sibutramine hydrochloride monohydrate. Thus, in one embodiment, the anti-obesity agent is a monoamine reuptake inhibitor, and pharmaceutically acceptable salts or esters thereof. In a subclass of this class, the monoamine reuptake inhibitor is selected from sibutramine, and pharmaceutically acceptable salts and esters thereof.

Phytopharm compound 57 is also known as CP 644,673. Thus, in one embodiment, the anti-dyslipidemic agent is Phytopharm compound 57.

Serotonin reuptake inhibitors, and releasers, useful in the present invention include: dexfenfluramine, fluoxetine, and other serotonin reuptake inhibitors, including, but not limited to, those in U.S. Patent No. 6,365,633; and PCT Patent Application Nos. WO 01/27060, and WO 011162341. Thus, in one embodiment, the anti-dyslipidemic agent is a Serotonin reuptake inhibitors or releaser.

11β HSD-1 inhibitor useful in the present invention include, but are not limited to, BVT 3498, BVT 2733, and those compounds disclosed in WO 01190091, WO 01190090, WO 01190092, which are incorporated by reference herein in their entirety. Thus, in one embodiment, the anti-dyslipidemic agent is a 11β HSD-1 inhibitor.

Uncoupling Protein (UCP-1, UCP-2, and UCP-3) activators useful in the present invention include: PCT Patent Application No. WO 99/00123. Specific uncoupling protein (UCP-1, UCP-2, and UCP-3) activators useful in the present invention include, but are not limited to, phytanic acid, 4-((E)-2 (5,6,7,8-tetrahydro-5,5,8,8-tetramethyl-2-napthalenyl)-1-propenyl]benzoic acid (TTNPB), and retinoic acid. Thus, in one embodiment, the anti-dyslipidemic agent is a UCP-1, UCP-2, or UCP-3 activator.

β3 adrenergic receptor (β3) agonists useful in the present invention include: US Patent Application Nos. 5,705,515, and US 5,451677; and PCT Patent Application Nos. WO 01/74782, and WO 02/32897. Specific, β3 agonists useful in the present invention include, but are not limited to, AD9677/TAK677 (Dainippon/ Takeda), CL-316,243, SB 418790, BRL-37344, L- 796568, BMS-196085, BRL-35 135A, CGP12177A, BTA-243, GW 427353, Trecadrine, Zeneca D7114, and SR 591 19A. Thus, in one embodiment, the anti-dyslipidemic agent is a β3 adrenergic receptor β3) agonist.

Thyroid hormone β agonists useful in the present invention include: PCT Application No. WO 02/15845; and Japanese Patent Application No. JP 2000256190. Specific thyroid hormone β agonists useful in the present invention include, but are not limited to, KB-2611 (KaroBioBMS). Thus, in one embodiment, the anti-dyslipidemic agent is a Thyroid hormone agonist.

Specific fatty acid synthase (FAS) inhibitors useful in the present invention, include, but are not limited to, Cerulenin and C75. Thus, in one embodiment, the anti-dyslipidemic agent is a FAS inhibitor.

Specific phosphodieterase (PDE) inhibitors useful in the present invention, include, but are not limited to, theophylline, pentoxifylline, zaprinast, sildenafil, amrinone, milrinone, cilostamide, rolipram, and cilomilast. Thus, in one embodiment, the anti-dyslipidemic agent is a

Lipase inhibitors useful in the present invention include, but are not limited to, those disclosed in PCT Application No. WO 01/77094, and U.S. Patent Nos. 4,598,089. 4,452,813, 5,512,565, 5,391,571, 5,602,151, 4,405, 644, 4,189,438, and 4,242,453. Specific lipase inhibitors useful in the present invention include, but are not limited to, tetrahydrolipstatin (orlistat/Xenical), Triton WR1339, RHC80267, lipstatin, teasaponin, and diethylumbelliferyl phosphate, FL-386, WAY-121898, Bay-N-3176, valilactone, esteracin, ebelactone A, ebelactone B. RHC 80267, stereoisomers thereof, and pharmaceutically acceptable salts of said compounds and stereoisomers. Thus, in one embodiment, the anti-obesity agent is a lipase inhibitor, and pharmaceutically acceptable salts or esters thereof. In a subclass of this embodiment, the lipase inhibitor is orlistat, and the pharmaceutically acceptable salts thereof.

Topiramate (Topimax), indicated as an anti-convulsant and an anti- convulsant, has been shown to increase weight loss. Thus, in one embodiment, the anti-dyslipidemic agent is Topiramate (Topimax).

Zonisamide (Zonegran), indicated as an anti-epileptic, has been shown to lead to weight loss. Thus, in one embodiment, the anti-dyslipidemic agent is Zonisamide (Zonegran).

The above compounds are only illustrative of the anti-obesity agents and anti-dyslipidemic agents that can be used in the compositions of the present invention. As this listing of compounds is not meant to be comprehensive, the methods of the present invention may employ any anti- obesity agent and any anti-dyslipidemic agent, and are not limited to any particular structural class of compounds.

As indicated herein above, the compounds to be combined may be present as their pharmaceutically acceptable salts. If these compounds have, e.g., at least one basic center such as an amino group, they can form acid addition salts thereof. Similarly, the compounds having at least one acid group (for example COOH) can form salts with bases. Corresponding internal salts may furthermore be formed, if a compound comprises, e.g., both a carboxy and an amino group.

The corresponding active ingredients or a pharmaceutically acceptable salts may also be used in form of a solvate, such as a hydrate or including other solvents used, e.g., in their crystallization.

### Pharmaceutical compositions

Furthermore, the present invention provides pharmaceutical compositions comprising a renin inhibitor, or a pharmaceutically acceptable salt thereof, and at least one therapeutic agent selected from the group consisting of (a) and (b) as defined above and a pharmaceutically acceptable carrier.

A pharmaceutical composition according to the present invention may be employed for the prevention of, delay the onset of or treatment of dyslipidemia, dyslipidemia associated with obesity, dyslipidemia-related disorders, obesity, obesity-related disorders and/or a disease or a condition modulated by the inhibition of renin activity.

As disclosed herein above, a renin inhibitor, in particular, aliskiren, preferably in the form of the hemi-fumarate salt thereof, and at least one therapeutic agent selected from the group consisting of (a) and (b) as defined above, may be co-administered as a pharmaceutical composition. The components may be administered together in any conventional dosage form, usually also together with a pharmaceutically acceptable carrier or diluent.

The pharmaceutical compositions according to the invention are those suitable for enteral, such as oral or rectal, transdermal and parenteral administration to mammals, including man. For oral administration the pharmaceutical composition comprising a renin inhibitor, in particular, aliskiren, preferably in the form of the hemi-fumarate salt thereof, and at least one therapeutic agent selected from the group consisting of (a) and (b) as defined above, can take the form of solutions, suspensions, tablets, pills, capsules, powders, microemulsions, unit dose packets and the like. Preferred are tablets and gelatin capsules comprising the active ingredient together with: a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired d) disintegrants, e.g., starches, agar, alginic acid or its sodium salt, or effervescent mixtures; and/or e) absorbants, colorants, flavors and sweeteners. Injectable compositions are preferably aqueous isotonic solutions or suspensions, and suppositories are advantageously prepared from fatty emulsions or suspensions.

Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-90%, preferably about 1-80%, of the active ingredient.

The pharmaceutical composition according to the present invention as described hereinbefore and hereinafter may be used for simultaneous use or sequential use in any order, for separate use or as a fixed combination.

The dosage of the active ingredients can depend on a variety of factors, such as mode of administration, homeothermic species, age and/or individual condition.

Normally, in the case of oral administration, an approximate daily dose of from about 1 mg to about 360 mg is to be estimated e.g. for a patient of approximately 75 kg in weight.

For example, the doses of aliskiren to be administered to warm-blooded animals, including man, of approximately 75 kg body weight, especially the doses effective for the inhibition of renin activity, e.g., in lowering blood pressure, are from about 3 mg to about 3 g, preferably from about 10 mg to about 1 g, e.g., from 20 to 200 mg/person/day, divided preferably into 1 to 4 single doses which may, e.g., be of the same size. Usually, children receive about half of the adult dose. The dose necessary for each individual can be monitored, e.g., by measuring the serum concentration of the active ingredient, and adjusted to an optimum level. Single doses comprise, e.g., 75 mg, 150 mg or 300 mg per adult patient.

In general, for treating, controlling, and/or preventing dyslipidemia, dyslipidemia associated with obesity, a dyslipidemia-related disorder, obesity and an obesity-related disorder, the anti-obesity agent in the combination is administered at a daily dosage of from about 0.0001 mg/kg to about 100 mg/kg of body weight, preferably from about 0.001 mg/kg to about 50 mg/kg, given in a single dose or in divided doses two to six times per day, or in sustained release form; and the anti-dyslipidemic agent in the combination is administered at a daily dosage of from about 0.0001 mg/kg to about 100 mg/kg of body weight, preferably from about 0. 001 mg/kg to about 50 mg/kg, given in a single dose or in divided doses two to six times per day, or in sustained release form. The dosage regimen may be adjusted to provide the optimal therapeutic response.

In general, for treating, controlling, and/or preventing cardiac hypertrophy, including left ventricular hypertrophy, the anti-obesity agent in the combination is administered at a daily dosage of from about 0.0001 mg/kg to about 100 mg/kg of body weight, preferably from about 0. 001 mg/kg to about 50 mg/kg, given in a single dose or in divided doses two to six times per day, or in sustained release form; and the anti-dyslipidemic agent in the combination is administered at a daily dosage of from about 0.0001 mg/kg to about 100 mg/kg of body weight, preferably from about 0.001 mg/kg to about 50 mg/kg, given in a single dose or in divided doses two to six times per day, or in sustained release form. The dosage regimen may be adjusted to provide the optimal therapeutic response.

In general, for treating, controlling, and/or preventing metabolic syndrome, the anti-obesity agent, the anti-dyslipidemic agent, the anti- hypertensive agent, and the anti-diabetic agent in the given combination are administered at a daily dosage of from about 0.0001 mg/kg to about 100 mg/kg of body weight, preferably from about 0.001 mg/kg to about 50 mg/kg, given in a single dose or in divided doses two to six times per day, or in sustained release form. The dosage regimen may be adjusted to provide the optimal therapeutic response.

The compounds of this invention can be administered to humans in the dosage ranges specific for each compound.

Leptin may be administered at a daily dosage of from about 0.01 mg/kg to about 20 mg/kg, preferably, from about 0.01 mg/kg to about 0.3 mg/kg, preferably injected in a single dose or in divided doses.

Nalmefene may be administered at a daily dosage of from about 0.0001 mg/kg to about 10 1 mg/kg, preferably from about 0.001 to about 0.05 mg/kg.

Orlistat may be administered at a daily dosage of from about 20 mg to about 1200 ma, preferably from about 120 mg to 400 mg in a single dose or divided doses 2 to 3 times per day or in sustained release form; more preferably a 120 mg single dose 3 times per day, or in sustained release form.

Sibutramine may be administered at a daily dosage of from about 0.01 mg/kg to about 10 mg/kg, preferably from about 0.01 mg/kg to about 1 mg/kg in a single dose or in divided doses 2 to 3 times per day, or in sustained release form; more preferably the single daily dose of sibutramine is 5 ma, 10 ma, 15 ma, 20 mg or 30 mg orally.

Topiramate (Topamax) may be administered at a daily dosage of from about 10 mg to about 1,600 mg per day, preferably from about 50 mg to about 400 mg per day in a single dose or in divided doses, or in sustained release form.

Specific dosages for commercially available anti-obesity and anti-dyslipidemic agents which are useful in the present invention may be found in the Physician's Desk Reference, Edition 56 (2002).

The effective dosage of each of the active ingredients employed in the composition may vary depending on the particular compound employed, the mode of administration, the condition being treated and the severity of the condition being treated. Thus, the dosage regimen utilizing the compositions of the present invention is selected in accordance with a variety of factors including type, species, age, general health, body weight, diet, sex and medical condition of the subject; the severity of the condition to be treated; the renal and hepatic function of the patient; the drug combination; and the particular compounds employed and their routes of administration. A physician, clinician or veterinarian of ordinary skill can readily determine and prescribe the effective amount of the drug required to prevent, counter or arrest the progress of the condition.

Compositions of the agents in the combinations of the present invention (e.g. the anti-obesity agent; the anti-dyslipidemic agent) will generally also be within the aforementioned range, but in each case, an effective dose of each active ingredient should be used.

### Methods and Uses

Another aspect of the present invention relates to a method for the prevention, delay the onset of or treatment of dyslipidemia, dyslipidemia associated with obesity, dyslipidemia-related disorders, obesity, obesity-related disorders and/or a disease or a condition modulated by the inhibition of renin activity comprising administering to a warm-blooded animal, including man, in need thereof, a therapeutically effective amount of a combination comprising a renin inhibitor, in particular, aliskiren, or a pharmaceutically acceptable salt thereof, and at least one therapeutic agent selected from the group consisting of (a) and (b) as set forth above.

Accordingly, the combination according to the present invention may be used, e.g., for the prevention, delay the onset of or treatment of dyslipidemia, dyslipidemia associated with obesity, dyslipidemia-related disorders, obesity, obesity-related disorders and/or a disease or a condition modulated by the inhibition of renin activity. Especially, the combination according to the present invention may be used, e.g., for the prevention of, delay the onset of or the treatment of diseases and conditions selected from the group consisting of
(a) hypertension, congestive heart failure, renal failure, especially chronic renal failure, coronary and vascular restenosis, e.g. restenosis after percutaneous transluminal angioplasty, and restenosis after coronary artery bypass surgery;
(b) atherosclerosis, eg., due to a reduction in oxidant stress, a direct effect on lipids or to an anti-inflammatory effect of one or all components of the combination,
(c) insulin resistance and syndrome X/metabolic syndrome, diabetes mellitus type 2, obesity, nephropathy, e.g. diabetic nephropathy, renal failure, e.g. chronic renal failure, hypothyroidism, survival post myocardial infarction (MI), coronary heart diseases, hypertension in the elderly, familial dyslipidemic hypertension, increase in the formation of collagen, fibrosis, eg., cardiac, renal or liver, remodeling (vascular) following hypertension and/or hyperlipidemia (antiproliferative effect of the combination which may be dependent or independent of an action on lipids), and vascular remodeling which may be, in part, due to an anti-inflammatory effect and all these diseases or conditions associated with or without hypertension;
(d) endothelial dysfunction with or without hypertension,
(e) hyperlipidemia including Fredrickson types III hyperlipidemia, hyperlipoproteinemia, hypertriglyceridemia, hypercholesterolemia, low high density lipoprotein cholesterol levels, high low density lipoprotein cholesterol, atherosclerosis and hypercholesterolemia, heart attack, stroke, obesity, and left ventricular hypertrophy
(f) glaucoma
(g) isolated systolic hypertension (ISH),
(h) diabetic retinopathy
(i) peripheral vascular disease.

Preferably, said combination may be used for the treatment of hypertension, including ISH, as well as congestive heart failure, metabolic syndrome, endothelial dysfunction, impaired vascular compliance, diabetes especially type 2 diabetes mellitus, hypertensive or non-hypertensive nephropathy, IgA nephropathy, hyperlipidemia, hypertriglyceridemia, hypercholesterolemia, low high density lipoprotein cholesterol levels, high low density lipoprotein cholesterol levels, atherosclerosis, coronary artery disease, heart attack, and stroke, obesity, and left ventricular hypertrophy. More preferably said combination may be used for the treatment of hypertension, hyperlipidemia, hypertriglyceridemia, hypercholesterolemia, low high density lipoprotein cholesterol levels, high low density lipoprotein cholesterol levels, atherosclerosis, obesity, and left ventricular hypertrophy.

Preferably, the combined therapeutically effective amounts of the active agents according to the combination of the present invention can be administered simultaneously or sequentially in any order, e.g., separately or in a fixed combination.

Additionally, the present invention relates to the use of a combination comprising a renin inhibitor, in particular, aliskiren, or a pharmaceutically acceptable salt thereof, and at least one therapeutic agent selected from the group consisting of (a) and (b) as forth above and a pharmaceutically acceptable carrier; for the manufacture of a medicament for the prevention, delay the onset of or treatment of dyslipidemia, dyslipidemia associated with obesity, dyslipidemia-related disorders, obesity, obesity-related disorders and/or a disease or a condition modulated by the inhibition of renin activity.

Preferred are combinations, such as a combined preparations or pharmaceutical compositions, respectively, comprising aliskiren, or a pharmaceutically accepted salt thereof, and at least one therapeutic agent selected from the group consisting of (a) the afore-mentioned anti-dyslipidemic agent and (b) the afore-mentioned anti-obesity agent. The examples, including the most preferred embodiments, of the afore-mentioned anti-dyslipidemic agent and the afore-mentioned anti-obesity agent are as set forth above.

Particulary preferred anti-dyslipidemic agents to be combined with the renin inhibitor, in particular, aliskiren, are selected from the group consisting of cholestyramine, colesevelem, colestipol (Colestid), LoCholest; Questran, beta-sitosterol, ezetimibe, tiqueside, avasimibe, eflucimibe, KY505 SMP 797, probucol, GW 4064, SR 103912, GW 3965, T9013137, XTCO179628, niacin , nicotinic acid, BARI 1453, SC435, PHA384640, S8921, AZD7706, inplitapide, LAB687, and CP346086, more preferably cholestyramide, -sitosterol, ezetimibe, tiqueside, avasimibe, eflucimibe, KY505 SMP 797, probucol, niacin and nicotinic acid, most preferably ezetimibe, cholestyramide and niacin.

Particulary preferred anti-obesity agents to be combined with the renin inhibitor, in particular, aliskiren are selected from the group consisting of paroxetine, fluoxetine, fenfluramine, fluvoxamine, sertraline, imipramine, GW 320659, despiramine, talsupram, nomifensine, thioperamide, clobenpropit, iodophenpropit, imoproxifan, GT2394 (Gliatech), leptin, nalmefene (Revex), 3-methoxynaltrexone, naloxone, naltrexone, axokine (Regeneron), butabindide, sibutramine, dexfenfluramine, fluoxetine, theophylline, pentoxifylline, zaprinast, sildenafil, amrinone, milrinone, cilostamide, rolipram, cilomilast, tetrahydrolipstatin (orlistat/Xenical), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC 80267, topiramate, and zonisamide, most preferably sibutramine and orlistat.

Since the present invention has an aspect that relates to methods for the prevention of, delay the onset of or treatment with a combination of compounds which may be administered separately, the invention also relates to combining separate pharmaceutical compositions in a kit form. Accordingly, the pharmaceutical composition according to the present invention may comprise a "kit of parts" in the sense that the components can be dosed independently or by use of different fixed combinations with distinguished amounts of the components at different time points. The parts of the "kit of parts" can then, e.g., be administered simultaneously or chronologically staggered, that is at different time points and with equal or different time intervals for any part of the "kit of parts". Preferably, the time intervals are chosen such that the effect on the treated disease or condition in the combined use of the parts is larger than the effect that would be obtained by use of only any one of the components. Preferably, there is at least one beneficial effect, e.g., a mutual enhancing of the efficacy of a renin inhibitor, e.g., aliskiren, or a pharmaceutically acceptable salt thereof, and at least one therapeutic agent selected from the group consisting of (a) and (b) as defined above;in particular, a potentiation or a synergism, e.g., a more than additive effect, additional advantageous effects, less side effects, a combined therapeutical effect in a non-effective dosage of one or each of the components, especially, a potentiation or a strong synergism.

The term "potentiation" shall mean an increase of a corresponding pharmacological activity or therapeutical effect, respectively. Potentiation of one component of the combination according to the present invention by co-administration of another component according to the present invention means that an effect is being achieved that is greater than that achieved with one component alone:

The term "synergistic" shall mean that the drugs, when taken together, produce a total joint effect that is greater than the sum of the effects of each drug when taken alone.

The invention furthermore relates to a commercial package comprising the combination according to the present invention together with instructions for simultaneous, separate or sequential use.

The pharmaceutical activities as effected by administration of a renin inhibitor, in particular, aliskiren, or a combination of the active agents used according to the present invention can be demonstrated, e.g., by using corresponding pharmacological models known in the pertinent art. The person skilled in the pertinent art is fully enabled to select a relevant animal test model to prove the hereinbefore and hereinafter indicated therapeutic indications and beneficial effects.

To evaluate the antihypertensive activity of the combination according to the invention, for example, the methodology as described by Lovenberg W: Animal models for hypertension research. Prog. Clin. Biol. Res. 1987, 229, 225-240 may be applied. For the evaluation that the combination according to the present invention may be used for the treatment of congestive heart failure, for example, the methods as disclosed by Smith HJ, Nuttall A: Experimental models of heart failure. Cardiovasc Res 1985, 19, 181-186 may be applied. Molecular approaches such as transgenic methods are also described, for example by Luft et al.: Hypertension-induced end-organ damage, "A new transgemic approach for an old problem", Hypertension 1999, 33, 212-218.

At least three animal models are available to study the antihypertensive and anti-dyslipidemic effects of this combination. They include:
- Spontaneously hypertensive hyperlipidemic rats (SHHR) - Clin Exp Pharmacol Physio 2003 Aug 30 (8): 537-44
- Weanling Wistar rats (hypertensive and hypertriglyceridemic models) - Clin Exp Hypertens 2005 Jan; 27 (1): 45-47
- Transgenic hyperlipidemic-genetic hypertension rat model - Mol Med 2003 May-Aug 9 (5-8): 135-42.

In particular the following model may be employed:
Cholesterol lowering activity is determined in the rat as follows:
   Male Sprague-Dawley rats (230-250 g) (Taconic Farms) are maintained ad libitum on water and a high cholesterol diet (1.5% cholesterol and 0.5% cholic acid) for two weeks prior to and during the 7-day treatment period. Groups of animals are treated orally by gavage with the vehicle alone or with test compound for 7 consecutive days. After the last dose, animals are fasted for 18 hours and blood is collected. Blood samples are centrifuged at 2500 rpm for 10 minutes to prepare plasma for total cholesterol determination as well as LDL and HDL cholesterol concentrations. HDL values are determined after LDL/VLDL precipitation (Warnick and Albers, 1978). All samples are analyzed enzymatically for cholesterol with a diagnostic reagent kit (Sigma Chemical Co., St. Louis, MO). The analysis is performed on a Bio-Mek automated work station. LDL/VLDL fractions are precipitated in the following manner: 0.35 mL of plasma is aliquoted into Eppendorf tubes to which 12 µL of 2M manganese chloride, 11.2 µL of sodium heparin (Porcine Intestinal, 5000 units/mL), and 8.3 µL of normal saline are added. The samples are vortexed and are placed on ice for 15 minutes, then centrifuged at 4°C for 10 minutes at 1300 rpm and the supernatant is enzymatically analyzed for cholesterol. The HDL cholesterol concentration is adjusted for dilution by multiplying the supernatant cholesterol value by 1.09. LDL/VLDL cholesterol values are obtained by subtracting HDL cholesterol from total cholesterol.

The person skilled in the pertinent art is fully enabled to select a relevant test model to prove the efficacy of a combination of the present invention in the hereinbefore and hereinafter indicated therapeutic indications. Human renin inhibitors, for example aliskiren, typically have a high species specificity, most notably for human renin. Unexpectedly, aliskiren retains reasonable potency against dog and mouse renin but is 1-2 orders of magnitude weaker as a rat renin inhibitor. Consequently, rat models are not the preferred species but can be used if either higher doses of aliskiren (or another renin inhibitor) are used or if an animal species is used in which the RAS is particularly activated or artificially 'over-activated'. A relevant and useful model for evaluating the effects of a combination of a renin inhibitor with a drug to treat obesity is the dog fed a high fat diet to induce obesity. These animals are hypertensive and overweight and have a generalized disturbance of metabolic parameters (Hall JE, Brands MW, Dixon WN, Smith MJ Jr. Obesity-induced hypertension. Renal function and systemic hemodynamics. Hypertension 22:292-299, 1993). Some mouse models such as the Agouti Yellow Obese Mouse (Correia MLG, Haynes WG, Rahmouni K, Morgan DA, Sivitz WI, Mark AL. The concept of selective leptin resistance. Diabetes 51:439-442, 2002. ) or the transgenic mouse with reduced brown fat (Cittadini A, Mantzoros CS, Hampton TG, Travers KE, Katz SE, Morgan JP, Flier JS, Douglas PS. Cardiovascular abnormalities in transgenic mice with reduced brown fat. Circulation 100:2177-2183, 1999) can serve as useful models of human obesity with superimposed hypertension. A less preferred, but still useful animal model is the stroke prone spontaneously hypertensive rat (Izumo strain) crossed with Zucker fatty (ZF)(fa/fa) rats to yield the SHRSP fatty (fa/fa) rat (Hiraoka-Yamamoto et al., 2004). Additionally, Zucker rats (OZR or fa/fa) or its diabetic relative, the Zucker Diabetic Fatty Rat (ZDF) may also be utilized. These animal models are characterized by hypertension, obesity, insulin resistance/glucose intolerance (OZR) or diabetes (ZDF) and hyperlipidemia (Toblli JE, DeRosa G, Rivas C, Cao G, Piorno P, Pagan P, Forcada P. Cardiovascular protective role of a low-dose antihypertensive combination in obese Zucker rats. J Hypertens 21:611-620, 2003; van Zwieten PA. Diabetes and hypertension: experimental models for pharmacological studies. Clin Exp Hypertens 21:1-6, 1999, Mizuno M, Sada T, Kato M, Koike H. Renoprotective effects of blocakde of angiotensin II AT1 receptors in an animal model of type 2 diabetes. Hypertens Res 25(2):271-278, 2002). Although the effects of the combination can be evaluated in OZR of any age, the metabolic parameters will vary according to the age of the animal. Older animals may present with more substantial structural and functional changes than young rats due to the long-standing metabolic disturbance and the impact of these changes on the overall disease process. Therefore, results from laboratory to laboratory may vary due to the age at which the animals are used for study. Animals are typically 10 - 20 weeks of age when experiments are initiated. Typically, measurement of a wide variety of metabolic and functional parameters, including plasma lipids, plasma glucose, glucose tolerance, plasma insulin, body weight, and blood pressure, are made. Other more specific measurements may also be performed to assess endothelial function, oxidative stress, organ weight determinations, assessment of cardiac mass, cardiac and renal function and morphometric analyses. Refer to Zhou MS, Jaimes EA, Raij L. Atorvastatin prevents end-organ injury in salt-sensitive hypertension. Hypertens 44:186-190, 2004 for a description of some of these measurements. Blood pressure is also monitored chronically and with greater consistency using radiotelemetry as described below.

Radiotransmitters are implanted into either rats of at least 7 weeks of age or with body weight greater than 200 grams. In mice, body weight should exceed 20 grams at the time of telemetry implantation. Drug treatment can be initiated at any time following a two week recovery period from surgery. Drugs are administered once daily by oral gavage but may be given by other routes (eg., intra-peritoneal, intra-venous, or subcutaneous). Rats or mice are randomized to receive one of the various treatments, including a vehicle control. Drugs are administered by oral gavage, once daily in the morning for several weeks to 2 or 3 months. In special cases, drugs may be administered in the evening or multiple times per day. Also, for some studies in which the effects of feeding or behavior is to be noted, rats or mice can be placed on a reverse lighting schedule to induce a diurnal shift in eating and drinking patterns. Blood pressure (mean, systolic, and diastolic) and heart rate are continuously monitored, 24 hours per day for the full duration of the study using radiotelemetric procedures. All values depict 24 hour average responses for each animal but data summarization may also be performed using other time intervals, for example, hourly averaging. Body weights were recorded at weekly intervals or in some studies, may be monitored daily. Upon completion of the study, all rats or mice are sacrificed and hearts removed, sectioned and weighed. Cardiac mass was determined as the left ventricular weight to body weight ratio for each animal within a treatment group. Other tissues, including but not restricted to the kidney, may be removed at sacrifice for determination of biochemical markers, to assess the extent of tissue damage (histology, immunohistochemistry, etc), and for gene expression profiling. Blood sampling for measurement of glucose, insulin, lipids or other biochemical markers of metabolic function can be performed at various time points but is specifically limited (blood volume and frequency) depending upon the species. Thus, in a dog model, more frequent blood sampling and larger volumes are possible and consequently, a more extensive biochemical marker analysis can be performed.

### Test Example 1: in vitro CETP activity inhibitory effect in whole plasma

Plasma containing [³H] CE-HDL₃ (600,000 dpm/ml) was prepared by adding donor lipoprotein obtained in the above (1) to plasma from healthy subjects. A sample solution was prepared using a 1:1 solution of N-methylpyrrolidone and polyethyleneglycol 400 as a solvent. The sample solution or the solvent alone (2µl) and the plasma containing [³H] CE-HDL₃ (100 µl) were added to microtubes and incubated for 4 hours at 3°C or 4°C. After ice cooling, a TBS solution (20 mM Tris/0.15 M NaCl (pH 7.4)] containing 0.15 M magnesium chloride and 0.3 % dextran sulfate (100 µl) were added to each microtube and mixed well. After allowing the microtubes to stand at 4°C for 30 minutes, centrifugation (8,000 rpm, 4°C, 10 minutes) was conducted and the radioactivity of the resulting supernatant (HDL fraction) was determined with a scintillation counter. The difference between the values obtained after incubation at 4°C and 37°C with the solvent alone was regarded as CETP activity and a decrease (%) of the measured values produced by the samples was regarded as inhibition rate (%) of CETP activity. Based on the inhibition rate (%) of CETP activity, IC₅₀ value of each sample was calculated.

### Test Example 2: ex vivo CETP activity inhibitory effect of plasma from transgenic mice

Samples were suspended in a 0.5 % methylcellulose solution and administered orally using a plastic probe to transgenic mice having introduced human CETP gene (hereafter referred to as mice, prepared using the method described in Japanese Patent Application No. Hei 8-130660), which had been fasted overnight. Blood was collected before administration, and 6 hours after administration CETP activity in the plasma was determined using the following method.

Donor lipoprotein ([³H]CE-HDL₃, containing 0.21 µg cholesterol) obtained in the above (1), acceptor lipoprotein obtained in the above (2) (containing 21 µg of cholesterol), and 0.9 µg of mice plasma were added to microtubes. A total volume was adjusted to 600 µl/tube with a TBS solution [10 mM Tris/0.15 M NaCl (pH 7.4)]. The microtubes were incubated for 15 hours at 37°C or 4°C. Then, an ice-cooled TBS solution (400 µl/tube) and a 0.3 % dextran sulfate solution (100 µl/tube) containing 0.15 M magnesium chloride were added to the microtubes and mixed well. After allowing the microtubes to stand for 30 minutes at 4°C, centrifugation (8,000 rpm, 4°C, 10 minutes) was carried out and radioactivity of the resulting supernatant (HDL fraction) was determined with a scintillation counter. The difference between measured values obtained by incubating plasma of individual mice at 4°C and 37°C before administration of the samples were regarded as CETP activity and a decrease (%) of measured values after administration of samples was regarded as inhibition rate (%) of CETP activity.

Under certain circumstances, drugs with different mechanisms of action may be combined. However, just considering any combination of drugs having different modes of action but acting in the similar field does not necessarily lead to combinations with advantageous effects.

When employing the combination in accordance with the present invention, the following improvements are in particular noted:
- Essential hypertension could be treated more effectively;
- Reduction of elevated total-C, LDL-C, Apo B, and TG, an to increase HDL-C in patients with primary hypercholesterolemia (heterozygous familial and nonfamilial) and mixed dyslipidemia (Fredrickson types IIa and IIb);
- patients with hypertriglyceridemia (Fredrickson types IV hyperlipidemia) could be treated more effectively;
- patients with primary dysbetalipoproteinemia (Fredrickson types III hyperlipidemia) could be treated more effectively.
- Reduction of total-C and LDL-C in patients with homozygous familial hypercholesterolemia as an adjunct to other lipid-lowering treatments (e.g., LDL apheresis) or if such treatments are unavailable.

All the more surprising is the experimental finding that the combined administration of a renin inhibitor, in particular, aliskiren, and at least one therapeutic agent selected from the group consisting of (a) and (b) as defined above, or, in each case, a pharmaceutically acceptable form thereof, results not only in a beneficial, especially a potentiating or a synergistic therapeutic effect, but independent thereof, additional benefits resulting from combined treatment can be achieved such as a surprising prolongation of efficacy, decreased time necessary to achieve efficacy, a broader variety of therapeutic treatment and surprising beneficial effects on diseases and conditions associated with renin inhibiton. An additional and preferred aspect of the present invention is the prevention, delay the onset of and/or treatment of the condition of isolated systolic hypertension and impaired vascular compliance which means decreased vascular elasticity or arterial compliance.

Guidelines issued jointly by the European Society of Hypertension and the European Society of Cardiology as well as the seventh report of the Joint National Committee (JNC-7) on prevention, detection, evaluation and treatment of high blood pressure emphasize the need to achieve target blood pressure goals of < 140/90 mmHg in the general population and even lower levels of blood pressure for patients with diabetes and renal disease. Data from the National Health and Nutrition Examination Survey indicate that only 55 percent of hypertensive patients in the United States receive treatment, and only 29 percent are controlled at a blood pressure below 140/90 mmHg. Many reasons exist for inadequate blood pressure control including patient compliance, reluctance of physicians to titrate medications to their appropriate levels, concerns over adverse events, and treatment costs. New treatment options for physicians and patients may help to address some of these issues.

Isolated Systolic Hypertension (ISH) is the most common form of hypertension in the elderly. It is defined as elevated systolic blood pressure (above 140 mmHg) in conjunction with normal diastolic blood pressure (below 90 mmHg). Elevated systolic blood pressure is an independent risk factor for cardiovascular diseases and may lead e.g. to myocardial hypertrophy and heart failure. ISH is furthermore characterized by an increased pulse pressure, defined as the difference between systolic and diastolic blood pressures. Elevated pulse pressure is being recognized as the type of hypertension the least likely to be well controlled. A reduction of elevated systolic blood pressure and correspondingly of pulse pressure is associated with a significant risk reduction in cardiovascular death. It has surprisingly been found that the combination of a renin inhibitor, in particular, aliskiren, and at least one therapeutic agent selected from the group consisting of (a) and (b) as defined above, results in enhanced reduction of elevated diastolic and/or systolic blood pressure in patients with different forms of hypertension including ISH. This combination has also been found to reduce the pulse pressure both in hypertensive patients having type 2 diabetes mellitus and in hypertensive patients that do not have type 2 diabetes mellitus.

Furthermore, it has been found that the chronic co-administration of either a therapeutic agent selected from the group consisting of (a) or (b) as defined above, with a renin inhibitor, in particular, aliskiren, imparts the beneficial effect on blood vessel morphology and function and results in a decrease of vascular stiffness and correspondingly in a maintenance and in an improvement of vascular compliance, primarily arterial compliance.

Accordingly, it has been found that the addition of at least one therapeutic agent selected from the group consisting of (a) and (b) as defined above to that of a renin inhibitor, in particular, aliskiren, would potentiate the effect on systolic blood pressure and further improve vascular stiffness/compliance. Conversely, the proven antihypertensive effects of a renin inhibitor, in particular, aliskiren, on systolic and diastolic blood pressure may be potentiated by the addition of an insulin sensitizer and/or an insulin secretion enhancer. The benefit of these combinations may also extend to an additional or potentiated effect on endothelial function, and improvement of vascular function and structure in various organs/tissues including the kidney, heart, eye and brain. Through the reduction in glucose levels, an anti-thrombotic and anti-atherosclerotic effect can also be demonstrated. Reduction of glucose would prevent or minimize the glycosylation of any structural or functional protein within the cardio-renal system. This effect proves to be highly beneficial by evoking an additive or synergistic effect on vascular function/structure when administered with a renin inhibitor, in particular, aliskiren, which alone improves cardiovascular function and structure through a distinct mechanism.

Additionally, dyslipidemia and/or obesity may contribute, in part, to the development of diabetes, hypertension and atherosclerosis and other dyslipidemia and obesity related diseases. Combined administration of a renin inhibitor with either an therapeutic agent selected from the group consisting of (a) or (b) as defined above will evoke further antihypertensive effects, improve vascular dynamics in hypertensive patients to a greater extent than after administration of either agent given alone. Consequently, combined administration will simultaneously improve both the metabolic and cardiovascular abnormalities, two conditions that often coexist in patients.

Further benefits include the use of lower doses of the individual drugs to be combined according to the present invention, when compared to the doses used when administered alone, for example, that the dosages could not only often be lower but are also given less frequently, thereby reducing the incidence of side effects. This is in accordance with the desires and requirements of the patients to be treated.

For example, it has been observed that the combination according to the present invention provides benefit especially in the treatment of mild to moderate hypertension or isolated systolic hypertension in patients with dyslipidemia or obesity, regardless of their hypertensive status, e.g., by reducing the risk of negative cardiovascular events by two different modes of action.

For example, the renin inhibitor aliskiren in combination has proven to be useful in the treatment of dyslipidemia and/or obesity beyond the reduction of blood At sub-therapeutic doses, with respect to the treatment of hypertension, the combination according to the invention may be merely used for the treatment of dyslipidemia and/or obesity, especially hyperlipidemia, hypertriglyceridemia, hypercholesterolemia, low high density lipoprotein cholesterol levels, high low density lipoprotein cholesterol levels, atherosclerosis, coronary artery disease, heart attack, stroke and obesity. In view of the reduced dose of aliskiren, there is a considerable safety profile of the combination making it suitable for a first line therapy.

### Example 1:

### Composition of aliskiren 150 mg (free base) uncoated tablets in mg/unit.

| | **Roller compacted tablet** | **Dosage form 1** | **Dosage form 2** | **Dosage form 3** |
|---|---|---|---|---|
| **Component** | | | | |
| Aliskiren hemi-fumarate | 165.750 | 165.750 | 165.750 | 165.750 |
| Microcrystalline cellulose | 220.650 | 84.750 | 72.250 | 107.250 |
| Polyvinylpyrrolidon K 30 | - | - | 12.000 | 12.000 |
| rospovidone | 84.000 | 45.000 | 44.000 | 48.200 |
| Aerosil 200 | 4.800 | 1.500 | 1.500 | 1.800 |
| Magnesium stearate | 4.800 | 3.000 | 4.500 | 5.000 |
| **Total weight** | **480.000** | **300.000** | **300.000** | **340.000** |

### Composition of aliskiren 150 mg (free base) uncoated tablets in % by weight.

| | **Roller compacted tablet** | **Dosage form 1** | **Dosage form 2** | **Dosage form 3** |
|---|---|---|---|---|
| **Component** | | | | |
| Aliskiren hemi-fumarate | 34.53 | 55.25 | 55.25 | 48.75 |
| Microcrystalline cellulose | 45.97 | 28.25 | 24.08 | 31.545 |
| Polyvinylpyrrolidon K 30 | - | - | 4 | 3.53 |
| Crospovidone | 17.5 | 15 | 14.67 | 14.175 |
| Aerosil 200 | 1 | 0.5 | 0.5 | 0.53 |
| Magnesium stearate | 1 | 1 | 1.5 | 1.47 |
| **Total %** | **100.00** | **100.00** | **100.00** | **100.00** |

### Composition of aliskiren 150 mg (free base) uncoated tablets in mg/unit (divided into inner/outer phase).

| | | **Roller compacted tablet** | **Dosage form 1** | **Dosage form 2** | **Dosage form 3** |
|---|---|---|---|---|---|
| | **Component** | | | | |
| **Inner Phase** | Aliskiren hemi-fumarate | 165.75 | 165.75 | 165.75 | 165.75 |
| | Microcrystalline cellulose | 220.65 | 84.75. | 72.25 | 90.25 |
| | Polyvinylpyrrolidon K 30 | - | - | 12.00 | 12.00 |
| | Crospovidone | 36.00 | - | - | 14.20 |
| | Aerosil 200 | - | - | - | - |
| | Magnesium stearate | 2.40 | - | - | - |
| **Outer phase** | Crospovidone | 48.00 | 45.00 | 4.00 | 34.00 |
| | Microcrystalline cellulose | - | - | - | 17.00 |
| | Aerosil 200 | 4.80 | 1.50 | 1.50 | 1.80 |
| | Magnesium stearate | 2.40 | 3.00 | 4.50 | 5.00 |
| | **Total weight** | **480.00** | **300.00** | **300.00** | **340.00** |

### Composition of aliskiren 150 mg (free base) uncoated tablets in % by weight (divided into inner/outer phase).

| | | **Roller compacted tablet** | **Dosage form 1** | **Dosage form 2** | **Dosage form 3** |
|---|---|---|---|---|---|
| | **Component** | | | | |
| **Inner Phase** | Aliskiren hemi-fumarate | 34.53 | 55.25 | 55.25 | 48.75 |
| | Microcrystalline cellulose | 45.97 | 28.25 | 24.08 | 26.545 |
| | Polyvinylpyrrolidon K 30 | - | - | 4 | 3.530 |
| | Crospovidone | 7.5 | - | - | 4.175 |
| | Aerosil 200 | - | - | - | - |
| | Magnesium stearate | 0.5 | - | - | - |
| **Outer phase** | Crospovidone | 10 | 15 | 14.67 | 10 |
| | Microcrystalline cellulose | - | - | - | 5 |
| | Aerosil 200 | 1 | 0.5 | 0.5 | 0.53 |
| | Magnesium stearate | 0.5 | 1 | 1.5 | 1.47 |
| | Total % | 100.00 | 100.00 | 100.00 | 100.00 |

### Example 2:

### Composition of aliskiren (dosage form 3) film-coated tablets in mg/unit.

| **Dosage form 3 / Strength** | **75 mg (free base)** | **150 mg (free base)** | **300 mg (free base)** |
|---|---|---|---|
| **Component** | | | |
| Aliskiren hemi-fumarate | 82.875 | 165.750 | 331.500 |
| Microcrystalline cellulose | 53.625 | 107.250 | 214.500 |
| Polyvinylpyrrolidon K 30 | 6.000 | 12.000 | 24.000 |
| Crospovidone | 24.100 | 48.200 | 96.400 |
| Aerosil 200 | 0.900 | 1.800 | 3.600 |
| Magnesium stearate | 2.500 | 5.000 | 10.000 |
| **Total tablet weight** | **170.000** | **340.000** | **680.000** |
| Opadry premix white | 9.946 | 16.711 | 23.9616 |
| Opadry premix red | 0.024 | 0.238 | 1.8382 |
| Opadry premix black | 0.030 | 0.051 | 0.2002 |
| **Total fim-coated tablet weight** | **180.000** | **357.000** | **706.000** |

The dosages forms 1, 2 and 3 may be prepared, e.g., as follows:
1) mixing the active ingredient and additives and granulating said components with a granulation liquid;
2) drying a resulting granulate;
3) mixing the dried granulate with outer phase excipients;
4) compressing a resulting mixture to form a solid oral dosage as a core tablet; and
5) optionally coating a resulting core tablet to give a film-coated tablet.

The granulation liquid can be ethanol, a mixture of ethanol and water, a mixture of ethanol, water and isopropanol, or a solution of polyvinylpyrrolidones (PVP) in the before mentioned mixtures. A preferred mixture of ethanol and water ranges from about 50/50 to about 99/1 (% w/w), most preferrably it is about 94/6 (% w/w). A preferred mixture of ethanol, water and isopropanol ranges from about 45/45/5 to about 98/1/1 (% w/w/w), most preferably from about 88.5/5.5/6.0 to about 91.5/4.5/4.0 (% w/w/w). A preferred concentration of PVP in the above named mixtures ranges from about 5 to about 30% by weight, preferably from about 15 to about 25%, more preferably from about 16 to about 22%.

Attention is drawn to the numerous known methods of granulating, drying and mixing employed in the art, e.g., spray granulation in a fluidized bed, wet granulation in a high-shear mixer, melt granulation, drying in a fluidized-bed dryer, mixing in a free-fall or tumble blender, compressing into tablets on a single-punch or rotary tablet press.

The manufacturing of the granulate can be performed on standard equipment suitable for organic granulation processes. The manufacturing of the final blend and the compression of tablets can also be performed on standard equipment.

For example, step (1) may be carried out by a high-shear granulator, e.g., Collette Gral; step (2) may be conducted in a fluid-bed dryer; step (3) may be carried out by a free-fall mixer (e.g. container blender, tumble blender); and step (4) may be carried out using a dry compression method, e.g., a rotary tablet press.

## Claims

1. A combination comprising
a renin inhibitor, or a pharmaceutically acceptable salt thereof, and
at least one therapeutic agent selected from the group consisting of
(a) an anti-dyslipidemic agent selected from the group consisting of a bile acid sequesterant, a cholesterol absorption inhibitor, an acyl coenzyme A -cholesterol acyl transferase (ACAT) inhibitor, a squalene synthetase inhibitor, an anti-oxidant, a FXR receptor modulator, a LXR receptor agonist, a lipoprotein synthesis inhibitor, a microsomal triglyceride transport inhibitor, a bile acid reabsorption inhibitor, a triglyceride synthesis inhibitor, a transcription modulator, a squalene epoxidase inhibitor, a low density lipoprotein (LDL) receptor inducer, a 5-LO or FLAP inhibitor, and niacin or a niacin receptor agonist,
or a pharmaceutically acceptable salt thereof; and
(b) an anti-obesity agent selected from the group consisting of a 5HT (serotonin) transporter inhibitor, a NE (norepinephrine) transporter inhibitor, a ghrelin antibody, a ghrelin antagonist, a H3 (histamine H3) antagonist/inverse agonist, a MCH1 R (melanin concentrating hormone 1 R) antagonist, a MCH2R (melanin concentrating hormone 2R) agonist/antagonist, a NPY1 (neuropeptide Y Y1) antagonist, a NPY2 (neuropeptide Y Y2) agonist, a NPY5 (neuropeptide Y Y5) antagonist, leptin, a leptin derivative, an opioid antagonist, an orexin antagonist, a BRS3 (bombesin receptor subtype 3) agonist, a CCK-A (cholecystokinin-A) agonist, a CNTF (ciliary neurotrophic factor), a CNTF derivative, a GHS (growth hormone secretagogue receptor) agonist, 5HT2c (serotonin receptor 2c) agonist, a Mc3r (melanocortin 3 receptor) agonist, a Mc4r (melanocortin 4 receptor) agonist, a monoamine reuptake inhibitor, a serotonin reuptake inhibitor, topiramate, phytopharm compound 57, an ACC2 (acetyl-CoA carboxylase-2) inhibitor, a β3 (beta adrenergic receptor 3) agonist, a FAS (fatty acid synthase) inhibitor, a PDE (phosphodiesterase) inhibitor, a thyroid hormone,B agonist, an UCP-1 (uncoupling protein 1), 2, or 3 activator, an acyl-estrogen, a glucocorticoid antagonist, an 110 HSD-1 (11-beta hydroxy steroid debydrogenase type 1) inhibitor, a lipase inhibitor, a fatty acid transporter inhibitor, a dicarboxylate transporter inhibitor, a glucose transporter inhibitor, and a phosphate transporter inhibitor,
or a pharmaceutically acceptable salt thereof.

2. A combination according to Claim 1, wherein the anti-dyslipidemic agent is selected from the group consisting of enzetimbe, cholestyramide and niacin.

3. A combination according to Claim 1 or 2, wherein the anti-obesity agent is selected from the group consisting of sibutramine and orlistat.

4. A combination of at least two components selected from the group consisting of:
(i) a renin inhibitor or a renin inhibitor combined with a diuretic a pharmaceutically acceptable salt thereof; and
(ii) a cholesteryl ester transfer protein inhibitor or a pharmaceutically acceptable salt thereof.

5. A combination as claimed in claim 1, wherein the renin inhibitor is RO 66-1132 or RO-66-1168 of formulae 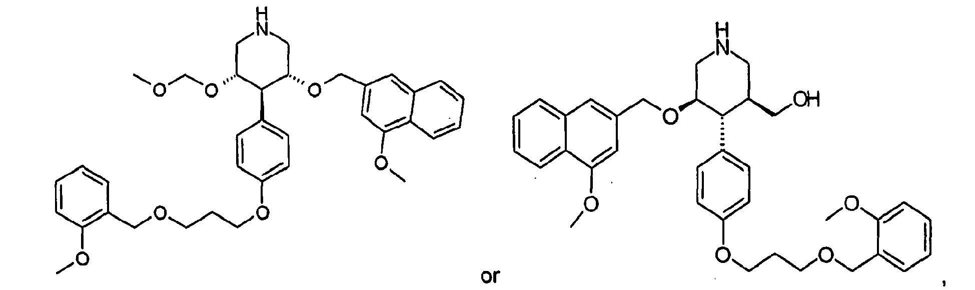 respectively, or is aliskiren or, in each case, a pharmaceutically acceptable salt thereof, wherein the diuretic is hydrochlorothiazide and wherein the CETP inhibitor is selected from the group consisting of JTT 705 of formula 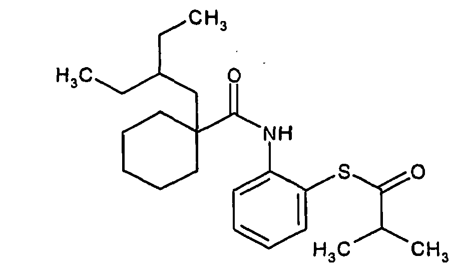 and
torcetrapib of formula

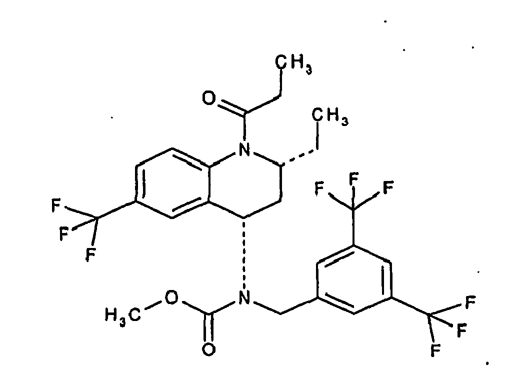

6. A combination according to any of Claims 1 to 5, wherein a renin inhibitor is selected from the group consisting of RO 66-1132, RO 66-1168 and a compound of the formula 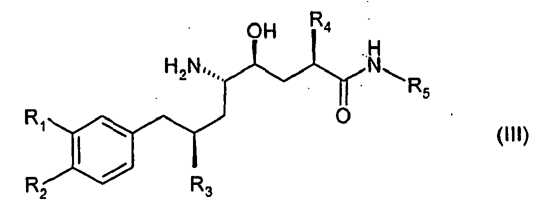 wherein R₁ is halogen, C₁₋₆halogenalkyl, C₁₋₆alkoxy-C₁₋₆alkyloxy or C₁₋₆alkoxy-C₁₋₆alkyl; R₂ is halogen, C₁₋₄alkyl or C₁₋₄alkoxy; R₃ and R₄ are independently branched C₃₋₆alkyl; and R₅ is cycloalkyl, C₁₋₆alkyl, C₁₋₆hydroxyalkyl, C₁₋₆alkoxy-C₁₋₆alkyl, C₁₋₆alkanoyloxy-C₁₋₆alkyl, C₁₋₆aminoalkyl, C₁₋₆alkylamino-C₁₋₆alkyl, C₁₋₆dialkylamino-C₁₋₆alkyl, C₁₋₆alkanoylamino-C₁₋₆alkyl, HO(O)C-C₁₋₆alkyl, C₁₋₆alkyl-O-(O)C-C₁₋₆alkyl, H₂N-C(O)-C₁₋₆alkyl, C₁₋₆alkyl-HN-C(O)-C₁₋₆alkyl or (C₁₋₆alkyl)₂N-C(O)-C₁₋₆alkyl; or a pharmaceutically acceptable salt thereof.

7. A combination according to Claim 6, wherein a renin inhibitor is a compound of formula (III) having the formula 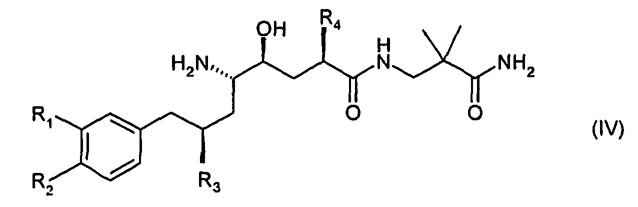 wherein R₁ is 3-methoxypropyloxy; R₂ is methoxy; and R₃ and R₄ are isopropyl; or a pharmaceutically acceptable salt thereof.

8. A combination according to Claim 7, wherein the compound of formula (IV) is in the form of the hemi-fumarate salt thereof.

9. A combination according to any of the preceding claims wherein the renin inhibitor is aliskiren and the anti-dyslipidemic agent is enzetimibe.

10. A combination according to any of the preceding claims wherein the renin inhibitor is aliskiren and the anti-dyslipidemic agent is cholestyramine.

11. A combination according to any of the preceding claims wherein the renin inhibitor is aliskiren and the anti-dyslipidemic agent is niacin.

12. A combination according to any of the preceding claims wherein (i) the renin inhibitor is aliskiren and the anti-obesity agent is sibutramine.

13. A combination according to any of the preceding claims wherein (i) the renin inhibitor is aliskiren and the anti-obesity agent is orlistat.

14. A pharmaceutical composition comprising the combination according to any of claims 1 to 11 and a pharmaceutically acceptable carrier.

15. A kit of parts comprising the combination of any of the preceding claims in the form of two or three separate units of the components.

16. A method of treatment and/or prevention of cardiovascular disorders modulated by the inhibition of renin activity comprising administering a therapeutically effective amount of the combination according to any of claims 1 to 13 to a mammal in need of such treatment.

17. A method of treatment and/or prevention of dyslipidemia, dyslipidemia associated with obesity, dyslipidemia-related disorders, obesity, and obesity-related disorders comprising administering a therapeutically effective amount of the combination according to any of claims 1 to 13 to a mammal in need of such treatment.

18. A method according to claim 16 or 17 wherein the diseases and conditions are selected from the group consisting of
(a) hypertension, congestive heart failure, renal failure, especially chronic renal failure, coronary and vascular restenosis, e.g. restenosis after percutaneous transluminal angioplasty, and restenosis after coronary artery bypass surgery;
(b) atherosclerosis, eg., due to a reduction in oxidant stress, a direct effect on lipids or to an anti-inflammatory effect of one or all components of the combination,
(c) insulin resistance and syndrome X/metabolic syndrome, diabetes mellitus type 2, obesity, nephropathy, e.g. diabetic nephropathy, renal failure, e.g. chronic renal failure, hypothyroidism, survival post myocardial infarction (MI), coronary heart diseases, hypertension in the elderly, familial dyslipidemic hypertension, increase in the formation of collagen, fibrosis, eg., cardiac, renal or liver, remodeling (vascular) following hypertension and/or hyperlipidemia (antiproliferative effect of the combination which may be dependent or independent of an action on lipids), and vascular remodeling which may be, in part, due to an anti-inflammatory effect and all these diseases or conditions associated with or without hypertension;
(d) endothelial dysfunction with or without hypertension,
(e) hyperlipidemia, hyperlipoproteinemia, hypertriglyceridemia, hypercholesterolemia, low high density lipoprotein cholesterol levels, high low density lipoprotein cholesterol, atherosclerosis and hypercholesterolemia, heart attack, stroke, obesity, and left ventricular hypertrophy
(f) glaucoma
(g) isolated systolic hypertension (ISH),
(h) diabetic retinopathy
(i) peripheral vascular disease.

19. A method according to any of claims 15 to 18 wherein the diseases and conditions are selected from the group consisting of hypertension, hyperlipidemia, hypertriglyceridemia, hypercholesterolemia, low high density lipoprotein cholesterol levels, high low density lipoprotein cholesterol levels, atherosclerosis, obesity, and left ventricular hypertrophy.
